# EUROPEAN PATENT SPECIFICATION

(11) **EP 2 575 905 B1**
(45) Date of publication and mention of the grant of the patent: **30.05.2018**
(21) Application number: 11721057.5
(22) Date of filing: 24.05.2011
(51) Int. Cl.: A61L 15/64, A61L 27/56, A61L 27/58, A61L 27/60, A61L 27/20, A61L 27/48, A61F 13/00, A61L 15/28, A61L 15/42

(54) **CHITOSAN BIOMIMETIC SCAFFOLDS AND METHODS FOR PREPARING THE SAME**
CHITOSANBASIERTE BIOMIMETISCHE GERÜSTE UND VERFAHREN ZU DEREN HERSTELLUNG
SUPPORTS BIOMIMÉTIQUES À BASE DE CHITOSANE ET LEURS PROCÉDÉS DE PRÉPARATION

(30) Priority: 04.06.2010 EP 10164959
(43) Date of publication of application: 10.04.2013
(73) Proprietor: Université de Liège, 4031 Angleur (Liege) (BE)
(72) Inventor: FILEE, Patrice, B-4020 Jupille-Sur-Meuse (BE); FREICHELS, Astrid, B-4801 Stembert (BE); JEROME, Christine, B-4102 Ougree (BE); AQIL, Abdelhafid, B-4020 Liege (BE); COLIGE, Alain, B-5100 Dave (BE); TCHEMTCHOUA TATEU, Victor, B-4031 Angleur (BE)
(86) International application number: PCT/EP2011/058455
(87) International publication number: WO 2011/151225

(56) References cited:
- WO-A2-2009/049565
- WO-A2-2010/040129
- FR-A1- 2 852 027
- US-A1- 2008 149 561
- US-A1- 2009 130 186

## Description

The invention concerns chitosan biomimetic scaffolds and methods for modulating their intrinsic properties such as rigidity, elasticity, resistance to mechanical stress, porosity, biodegradation and absorbance of exudates. Therefore, the present invention relates to a layered chitosan scaffold according to claim 1 comprising at least two fused layers, wherein at least one of the fused layer comprises a chitosan nanofiber membrane and the other fused layer comprises a chitosan sponge . Moreover, the present invention provides a layered chitosan scaffold characterized by (i) a good adhesion between the porous and nanofiber layers, (ii) a tuneable porosity of the nanofiber layer by tuning the distance between the nanofibers, (iii) a stable nanofibers and porous morphology even when immersed in water or other solvents and a process for the preparation of such layered chitosan scaffold. Finally, the present invention provides the layered chitosan scaffold of the invention or the layered chitosan scaffold produced by the process of the invention for use as a wound dressing, in regenerative medicine.

### FIELD OF THE INVENTION

This invention relates to the field of biopolymer development and, more particularly, to chitosan biomaterials. These biopolymers can be used mainly in tissue engineering as a scaffold for cell adhesion and proliferation, and, in particular, for tissue reconstruction. These biomaterials can be used for drug / vaccine/ cell delivery, for in vitro cell culture and cell differentiation, or in industrial processes using non-covalent immobilised enzymes.

### State of the art

### (i) Overview of chitosan

Chitosan is a wellknown cationic polysaccharide, poly-(1-4)-2-amino-2-deoxy-β-D glucose, obtained by partially N-deacetylation of chitin, the second most abundant polysaccharide in nature after cellulose. The molecular weight (MW) of chitosan can vary from 10 to over 1000 kDa (depending on the chitin source and deacetylation method) and the typical degree of deacetylation (DDA) ranges between 70% and 90%, allowing its solubilization in aqueous acidic solutions. Due to its reported biocompatibility, biodegradability, non-toxicity, antimicrobial, antifungal and wound healing properties, chitosan and its derivatives have been widely studied for use in the fields of medicine, cosmetics, agriculture, biochemical separation systems, tissue engineering and drug delivery systems. Chitosan has been processed in various forms, such as membranes, beads, films and fibers. In the recent years, special attention has been paid on the use of electrospinning for preparing chitosan nanofibrous membranes.

### (ii) Overview of electrospinning

Electrospinning is known from the art as an inexpensive, effective, and simple method for producing non-woven nanofibrous mats, which intrinsically have 10³ times larger specific surface to volume ratios, increased flexibility in surface functionalities, improved mechanical performances, and smaller pores than fibers produced using traditional methods . The necessary components of an electrospinning apparatus include a high power voltage supply, a capillary tube with a needle or pipette, and a collector that is usually composed of a conducting material . The collector is held at a relatively short distance from the capillary tube, which contains a polymeric solution connected to the high power supply. Hanging droplets of the polymer solution are initially held in place in the capillary tube due to surface tension. However, at a critical voltage, a conical protrusion, commonly referred to as a Taylor Cone, is formed . From this, a nearly straight jet emerges and travels for a few centimetres. While the jet is moving conically, it experiences bending instabilities and its field is directed toward the collector, which is oppositely charged. By the time the jet reaches the collector, the solvent evaporates, thus dry polymer fibers are deposited on the collector. All currently observed polymer jets have been theoretically and experimentally observed to be continuous; therefore, electrospinning creates seemingly endless ultrafine fibers that collect in a random pattern . The resulting mats (or membranes) from these small diameter fibers have very large surface area to volume ratios and small pore sizes. Fibrous mats are used in the aforementioned fields that chitosan could be used in. More specifically, because of the biofunctionality and biocompatibility of the biopolymer, electrospun chitosan could be used to improve tissue engineering scaffolds by increasing their cytocompatibility while also mimicking the native extracellular matrix. Incorporation of specific growth factors or catalytic proteins might also advantageously be used to favor cells regeneration.

Electrospinning of chitosan has proven to be difficult, pure chitosan fibres being obtained only from specific chitosan solutions such as in acetic acid or in trifluoroacetic acid (TFA) for example. Typically, blends of chitosan with another polymer, able to interfere with the strong interaction between the chitosan macromolecules and thus to improve its electrospinning ability, such as poly(ethylene oxide) (PEO), poly(vinyl alcohol) (PVA) or silk fibroin, have been used for the production of electrospun membranes .

### (iii) Healing of chronic wounds

The number of patients with chronic, poorly-healing wounds, such as diabetic foot, decubitus (ulcers) or wounds due to venous or arterial insufficiency, is on an upward trend, despite enormous advances in medicine. There are various well-known chemical and pharmaceutical preparations in use in the local treatment of wounds. These are available in various pharmaceutical forms such as ointments, gels, plasters, films, powders etc. It is clear from the diversity of wound-healing preparations on the market today that there is no one universal preparation. The following parameters are important in the development of a medical device such as this: maximization of adherence, moisture permeability control, infection control, safety, pain management, physical adaptability, stability in situ, cost-effectiveness, shelf stability, antibacterial effect, wound-healing effect, ease of handling (by surgeons, physicians, nurses and/or patients themselves) and, last but not least, comfort for patients by using, for example, dressing that can be detached from the wound without pain and without tearing the healing tissue.

Regardless of the type of wound and the extent of tissue loss, every wound healing process undergoes three overlapping but inseparable phases: inflammation, proliferation and remodeling. The immune system, mesenchymal cells and epidermal cells are of central importance in wound healing since they collaborate for the cleansing of the wound and the formation of a new granulation tissue through a complex network of interactions.

Although diffusible factors are indispensable for correct and sequentially ordered healing, adhesion molecules and mechanical stress factors are also of crucial importance, largely regulating the final properties of the repaired tissue. Finally, external and/or physical factors such as oxygen pressure, the relative wetness of the wound surface and the presence of pathogen also strongly influence the healing process.

### (iv) Dressings

### Wound dressing products

Biochemistry and histology of chitosan in wound healing is well-known. However, major limitations for its use as a wound dressing accelerating the healing of deep ulcer and potentially the tri-dimensional repair of connective tissues, are the facts that a chitosan film does not provide a tri-dimensional scaffold temporally replacing the lost tissue and that a chitosan sponge is a poor substrate for many cell types including keratinocytes, fibroblasts and endothelial cells. Moreover, due to its crustacean origin, anaphylactic reactions for some patients can not be totally excluded. Another limitation for commercialization of currently developed chitosan dressing results from their activation and/or stabilization through the use of potentially harmful chemicals such as cross-linkers for example.

### Physical properties of wound dressings

As reported above, an effective wound dressing not only protects the wound from its surroundings but must also possess various different properties related to its structure and its mechanical characteristics. It must provide an optimal microenvironment for healing, removing any excessive wound exudate and allowing oxygen to reach the surface of the wound, as examples. Mechanical properties of a dressing can also directly affect its adhesion to the wound surface and the behaviour of cell growing at its direct contact. For instance, pure chitosan produced as a film is not considered to be an appropriate wound dressing because of its poor tensile strength and elasticity. Thus, development of high strength composites that are biocompatible and that can help in wound healing may be necessary for wound healing applications

### (v) Biodegradation of chitosan

*In vitro* studies on the degradation of chitosan by oxidative-reductive depolymerization, by acid hydrolysis and by enzymes (lysozyme amylase, chitinase...) revealed a major dependency on the degree of acetylation (DDA). Completely de-acetylated chitosan shows very limited degradation by enzyme catalyzed hydrolysis while the extent of degradation and degradation kinetics increase with the extent of acetylation. Lysozyme is widely present in human body fluids (e.g. serum, saliva, tears) and is actively secreted by macrophages and neutrophils, and consequently the enzymatic degradation of chitosan by lysozyme has been studied in depth . Temperature, pH and ionic strength havebeen found to influence degradation kinetics with lysozyme

### (vi) Reacetylation of chitosan

As mentioned previously, *in vitro and in vivo* studies demonstrated that the biodegradation of chitosan was related to the degree of acetylation. Furthermore these studies clearly indicated that the biodegradation level is also related to the type of biopolymer (i.e., e.g, film, sponge, sponge, microbead, nanobead, microfiber, nanofiber, electrospun nanofiber, electrospun micro fibers) and to its morphology (i.e., e.g, multilayers, cross-linking between the fibers, porosity).
If re-acetylation of chitosan in chitin modifies the biodegradation level, this process can also alter the properties of the biopolymer. In function of the treatment, the morphology of the biopolymer can be drastically changed or altered . As a consequence, it is of crucial importance to develop standardized re-acetylation methods that permit to obtain biopolymers with the desired properties.

FR2852027 discloses multi-layered scaffolds from electrospun chitosan nanofibers.

### GOAL OF THE INVENTION

Novel biopolymers are desired that would support, enhance and improve cell adhesion and proliferation, tissue engineering and tissue reconstruction, in particular in the case of wound healing. Accordingly, the technical problem of the present invention is the provision of means and methods for tissue reconstruction based on biopolymer scaffolds.

### DESCRIPTION OF THE INVENTION

The technical problem is solved by the embodiments provided herein and as characterized in the claims. Specifically and in accordance with the present invention, a solution to this technical problem is achieved by providing a layered chitosan scaffold comprising at least two fused layers, wherein at least one of the fused layer comprises a chitosan nanofiber membrane and the other fused layer comprises a chitosan sponge. Moreover, the present invention provides a layered chitosan scaffold characterized by (i) a good adhesion between the porous and nanofiber layers, (ii) a tuneable porosity of the nanofiber layer by tuning the distance between the nanofibers, (iii) a stable nanofibers and porous morphology even when immersed in water or other solvents and a process for the preparation of such layered chitosan scaffold. The process for the preparation of a layered chitosan scaffold comprises the steps of: (a) preparing a solution of chitosan with a polymer improving its electrospining ability such as PEO ; (b) feeding the solution in a syringe; (c) covering a collector by a porous chitosan scaffold support layer; (d) applying an electrospinning voltage between the needle and said collector; and (e) collecting the layered chitosan scaffold. Finally, the present invention provides the use of the layered electrospun chitosan scaffold of the invention or the layered electrospun chitosan scaffold produced by the process of the invention as a wound dressing, in tissue engineering or for biomedical applications.

Accordingly, the heart of the present invention is notably the combination of two forms of chitosan (nanofibers and a sponge) and the modification of the entire device in order to improve its biological and mechanical properties.

In line with this, it was surprisingly found, as illustrated in the appended examples, that it was totally unexpected to demonstrate that endothelial cells, keratinocytes and fibroblasts (the three main cell types in the skin) adhere and proliferate on the layered chitosan nanofiber scaffold of the present invention. Moreover, the same scaffold stimulates the healing of excisional skin wounds in mice and, when inserted under the skin of mice, it does not induce any immune response but instead is invaded by healthy normal cells that remodel it and progressively replaced by endogenous macromolecules newly synthesized by invading cells, as it occurs physiological turnover of healthy tissues. Furthermore, the present invention provides for the first time electrospinning *on* chitosan sponges as well as the possibility of converting chitosan nanofibers into chitin nanofibers without altering their morphological characteristics, thereby enhancing biocompatibility or biodegradability in vitro and in vivo of the nanofibers as compared to chitosan nanofibers or in the form of films, sponges or granules. In contrast to the prior art, as described in detail below and illustrated in the appended examples, the layered scaffold is characterized (i) by a good adhesion between the sponge and the nanofiber layer insured by the fusion between nanofibers and sponge thanks to the described process, (ii) by a sponge/nanofiber morphology that resists immersion in aqueous media and other solvents except acids, (iii) by a tuneable porosity of the nanofiber layer allowing cells penetration. In addition, except for reacetylation, no chemical modification of the chitosan (e.g. no cross-linking with glutaraldehyde) is performed, keeping the chemical composition and structure of chitosane unmodified.

Furthermore, the present invention provides a procedure allowing to process the chitosan into nanofibers coated sponges without alteration of the chitosan chemical structure but with improved biological response of the non-woven mats modified sponge. This process includes mainly physical methods : (i) electrospinning for providing nanofibers of controlled diameter on chitosan sponge with partial fusion between the underlying mats and the nanofibers (ii) freeze-drying for regulating pore size, (iii) wetting in appropriate solvents or solutions for preserving nanofibers morphology e.g. in physiological conditions. Only the reconversion of chitosan into chitin needs a chemical reaction but that do not add chemical groups initially absent in chitosan (partial or total reacetylation of chitosan nanofibers in chitin nanofibers without altering their morphology). Importantly, the process allows stabilization of the entire scaffold by partial fusions between the two parts and consequently easy manipulation of the device without the use of cross-linking chemicals that are known to be toxic compounds and may induce immune reaction in patients. In addition, by total or partial reconversion of chitosan into chitin it is possible to finely regulate the biodegradability and the biological properties of the foreseen medical device.

Advantageously, the dressing of the present invention contains only chitosan, which is an abundant, well-defined and well-accepted biomaterial. In addition, among the produced and available chitosans on the market, chitosan from fungi origin, has been successfully tested which guarantees a better lot to lot reproducibility and traceability as compared to chitosan produced from crustacean waste of the fishing industry. This new dressing has been proved to accelerate wound healing processes in vitro in cell culture and in vivo in mice (as illustrated in the appended examples). It is easy to manipulate, favouring its use out of hospital and by patients themselves, and could be stored for years without altering its properties. Its price for a first series of prototype is 3 euros/cm2, and could be reduced in case of large scale production. It may still appear quite expensive but it is cheaper than other types of "bio-dressing". Moreover it would result in substantial reduction of the health expenses caused by the long-term treatment of chronic ulcer (several years of treatment, nurse expanses, amputation). As an additional advantage the new dressing provided herein could be used for treating animals since, by contrast to most of the new bio-dressings commercialized today, it does not contain products of human or animal origin that could induce immune response.

In summary, the present invention relates to methods for producing a biomimetic scaffold made of chitosan and to methods allowing to modulate its intrinsic properties such as its rigidity, elasticity, mechanical resistance, porosity, biodegradation and its capacity to absorb fluids in order to allow its use as dressing for the treatment of skin wounds, especially chronic ulcer. The invention is based also on unexpected results obtained in vitro and in vivo showing that electrospun nanofibers of chitosan have enhanced biological properties as compared to sponges, films or granule. These desirable unexpected properties for biomedical applications concern the behaviour of cells of the skin (keratinocytes, fibroblasts, endothelial cells) in culture and the improvement of the healing of full thickness skin wounds in mice.

### DETAILED DESCRIPTION OF THE INVENTION

The invention concerns chitosan scaffolds and methods for preparing and modifying the same, which are defined in the claims. The invention includes:
**(i)** Layered scaffolds preferably used for wound dressings comprising at least two fused layers, wherein at least one of the fused layer comprises corrugated chitosan nanofibers and the other support layer comprises porous chitosan. For instance the first layer which is in direct contact with the wound can be composed of a non woven mat of chitosan corrugated nanofibres. This layer serves as a scaffold for cell attachment and proliferation and favours tissue repair. The second layer, which mechanically supports the first layer, may have additional functions. Interactions between these two layers are strong enough for easy handling of the entire dressing but still loose enough for easy separation of the two layers if required for clinical applications. The second layer is composed of porous chitosan, as a sponge which is dedicated to absorb wound exudates and to improve mechanical property of the mat. The preparation method described for these layered scaffolds includes the possibility to control the porosity of the scaffold. Therefore, the scaffold of the invention can be effectively used as a support for triggering cell adhesion, growth and tissue repair and regeneration.

### Advantages of the embodiment (i) in regard to the state of art:

- Possibility to benefit simultaneously from the biological and/or mechanical and/or chemical properties of any individual layer or component.
- The invention permits an easy use and handling of chitosan nanofibers without requiring chemical cross-link and other harsh treatment that may alter the properties of the material or of any additives.
- Due to the progressive biodegradability of chitosan and to possibility to detach the first layer in contact to the wound from the upper layers, the described multi layers dressing can be remove from sticking or regenerating wounds without any tearing of newly formed tissues.

**(ii)** A combination of freeze-drying, wetting and electrospinning processes for preparing, without the use of toxic chemicals or toxic solvents, chitosan nanofibers mats that resist immersion in liquids (except acids) and that favour cell migration, adhesion, proliferation and invasion. At the end of the electrospinning and stabilization processes, this method permits, if desired, to obtain a nanofiber scaffold made of chitosan of preserved chemical structure.

### Advantages of the embodiment (ii) in regard to the state of art:

- Nanofibers mat of chitosane with fully preserved chemical structure and composition (MW, no cross-linking, acetylation degree,..) and of stable morphology towards temperature and aqueous or other solvents immersion obtained by a procedure without the use of toxic chemical, facilitating large scale exploitation in medicine and biology.
- Enhanced biological properties as demonstrated by in vitro and in vivo examples and data.
- Possibility to co-electrospin chitosan and additives without loosing the biological properties of additives such as enzymes or complex biological molecules

**(iii)** A chemical process which permits the reacetylation of electrospun chitosan nanofibers and without altering the fibrillary structure of the biomaterial. As electrospun chitosan nanofibers are poorly biodegradable, the reacetylation permits to restore sensitivity to human glycosyl hydrolases such as the human lysozyme and the human macrophage chitotriosidase. By controlling the reacetylation degree of chitosan, it is possible to develop biopolymers that, in function of the application, are more or less biodegradable. In addition, modulation of the reacetylation degree permits to combine the specific properties of chitosan and chitin in the same biopolymer. For instance, these properties are the tensile strength, elasticity of the nanofibers, air permeation, hydrophobicity.

### Advantages of the embodiment (iii) in regard to the state of art:

The chemical conversion of chitosan to chitin described in the invention offers different advantages:
- It does not alter the fibrillar structure of the nanofibers and the morphology of the electrospun chitosan biopolymer.
- As the processus of chemical conversion can be controlled, the acetylation rate of chitosan can be modulated. This feature is important because it allows restoring the susceptibility to human glycosylhydrolases and it permits to develop biopolymers that, in function of the application, are more or less biodegradable.
- In addition, modulation of the reacetylation rate permits to combine the specific properties of chitosan and chitin in the same biopolymer.
**Accordingly, the present invention provides the following:**
In accordance with the above, the present invention provides a layered chitosan scaffold, according to claim 1, comprising at least two fused layers, wherein at least one of the fused layers comprises a chitosan nanofiber membrane and the other fused layer comprises a porous chitosan support layer or sponge wherein the chitosan nanofiber membrane is electrospun onto the porous support layer.

In a preferred embodiment, the present invention provides a layered chitosan scaffold comprising at least two fused layers, wherein at least one of the fused layer consists of a chitosan nanofiber membrane and the other fused layer consists of a porous chitosan support layer or sponge.

The term "chitosan" is well-known in the art and relates to a cationic polysaccharide, that is obtained by partially N-deacetylation of chitin as already mentioned in the introductory part.

The term "sponge" relates to any materials having a porous and absorbent structure.

In the context of the present invention, the term "fused" relates to a partial or total adhesion between two materials.

In a preferred embodiment, the chitosan is from fungi origin. However, the present invention is not only bound to chitosan from fungi. Therefore, the chitosan may also be from other origins like crustacean.

In the context of the present invention, the term "scaffold" relates to a temporary three-dimensional frame containing pores and empty spaces that can be invaded and populated by cells or that can have various functions or properties such as, among others, absorption of wound exsudates. As described in detail below and shown in the appended examples, the layered scaffold comprises in a preferred embodiment two layers, i.e. a chitosan nanofiber membrane and a porous chitosan sponge. The chitosan nanofiber membrane is electrospun on the chitosan sponge. These two layers can be distinguished by to following features. Porous chitosan such as sponges are produced by freeze-drying of an acidic chitosan solution. They do not contain fibers but are rather constituted of layers and pillars having a thickness ranging from 1 to 100 microns (10⁻⁶ to 10⁻⁴ m) . The sizes of pores and empty spaces in the sponges are in the micrometric range. The thickness of the entire porous chitosan such as sponge may vary from 2 to 10 mm (210⁻³ to 10⁻² m). The electrospun membrane is made of chitosan nanofibres of a preferred diameter ranging from 50 to 300 nm (510⁻⁸ to 310⁻⁷m). Its thickness is below 1 mm(10⁻³m) and it contains pores in the nanometric range. In a preferred embodiment, the first layer is made of a non woven chitosan nanofiber mat obtained by electrospinning (first layer) on an absorbent neutralized chitosan sponge (chitosan produced by Kitozyme) or sponge from Hemcon (ChitoFlex™). In another preferred embodiment, the two layers of the layered chitosan scaffold are fused. This is because, when a chitosan solution is electrospun onto a dry chitosan supporting layer (that is a chitosan sponge) traces of solvent that are still in or on the nanofibers are abundant enough to locally swell the support which leads to partial or total fusion between the two layers (sponge and the nanofiber) upon drying.

The present invention is not only bound to the above-described two layered scaffold. In a preferred embodiment, the present invention provides for a scaffold of multiple sheets of layers. Accordingly, in a further preferred embodiment, there are provided scaffolds wherein the scaffold may comprise numerous layers of a chitosan nanofiber membrane and/or chitosan support layer. In context of the present invention, the porous chitosan support layer is described as chitosan sponge.

The support layer is an absorbent chitosan sponge either obtained by freeze-drying an acidic solution of chitosan (e.g. from fungi origin, as produced by Kitozyme) and then neutralizing the material by immersion in an alkaline solution, as 1 M NaOH for example, or purchased, as ChitoFlex™ sponge from Hemcon for example.

In a preferred embodiment, the layered chitosan scaffold of the present invention is a scaffold, wherein the scaffold is prepared by (a) preparing a sponge; (b) depositing chitosan nanofibers onto said chitosan sponge ; and (c) freeze-drying of the product resulting from step (b) for obtaining the layered chitosan scaffold.

The layered scaffold of the present invention is a scaffold, wherein the chitosan nanofiber membrane is produced by an electrospinning process. Hence, the layered scaffold of the present invention, is a scaffold, wherein one of the layers, the chitosan nanofiber membrane, is produced by an electrospinning process, said process comprising the steps of: (a) preparing a solution of chitosan with a polymer improving its electrospinning ability such as PEO (PolyEthylene Oxide); (b) feeding the solution in an injector, such as a syringe; (c) covering a collector by a porous chitosan support layer; (d) depositing, for example by applying an electrospinning voltage between the injector and said collector, chitosan nanofibres onto said porous chitosan support layer; and (e) collecting the layered chitosan scaffold.

In context of the present application, the term "electrospinning" is well known in the art and refers to a process as already mentioned in the introductory part above that uses an electrical charge to draw very fine (typically on the micro or nano scale) fibers from a liquid. The process is non-invasive and does not require the use of coagulation chemistry or high temperatures to produce solid threads from solution. This makes the process particularly suited to the production of fibres using large and complex molecules. Electrospinning from molten precursors is also practised; this method ensures that no solvent can be carried over into the final product. When a sufficiently high voltage is applied to a liquid droplet, the body of the liquid becomes charged, and electrostatic repulsion counteracts the surface tension and droplet is stretched, at a critical point a stream of liquid erupts from the surface. This point of eruption is known as the Taylor cone. If the molecular cohesion of the liquid is sufficiently high, stream breakup does not occur and a charged liquid jet is formed. As the jet (partially) dries in flight, the mode of current flow changes from ohmic to convective as the charge migrates to the surface of the fibre. The jet is then elongated by a whipping process caused by electrostatic repulsion initiated at small bends in the fibre, until it is finally deposited on the grounded collector. The standard setup for electrospinning consists of a spinneret (typically a hypodermic syringe needle) connected to a high-voltage (in general 5 to 50 kV) direct current power supply, a syringe pump, and a grounded collector. A polymer solution, sol-gel, particulate suspension or melt is loaded into the syringe and this liquid is extruded from the needle tip at a constant rate by a syringe pump. Alternatively, the droplet at the tip of the spinneret can be replenished by feeding from a header tank providing a constant feed pressure. This constant pressure type feed works better for lower viscosity feedstocks. The elongation and thinning of the fibre resulting from this bending instability lead to the formation of uniform fibres with nanometer-scale diameters. Solutions of 10.5% chitosan and 4% HMW PEO may be prepared by dissolving the appropriate amount of chitosan (in 6.5% acetic acid solution) and PEO (in distilled water), by stirring overnight. In another preferred embodiment, not only HMW PEO may be used but also any other natural or synthetic polymer with similar biophysical properties.
In this context, other natural or synthetic polymers are known to the person skilled in the art and the person skilled in the art is in a position to chose appropriate other natural or synthetic polymers which may comprise, inter alia, e.g polypolysaccharides such as hyaluronan, aliphatic polyesters, polyortoesters. The concentration of the solution of said HMW PEO or any other natural or synthetic polymer with similar biophysical properties may range from 0.01% to 40%. The concentration of the solution of said HMW PEO or any other natural or synthetic polymer with similar biophysical properties is 0.01% and 20%. As regards the concentration of the solution of chitosan it is not only envisaged to use a chitosan solution of 10.5%. Consequently. The concentration of the chitosan solution may range from 1% to 20%.
The next day, the chitosan and PEO solutions may then be mixed to obtain mixtures with weight ratios of chitosan: PEO of 90:10. Two ml of the well homogenized resulting mixture may be fed into 5ml plastic syringes fitted with blunt tipped stainless steel needles (gauge 18 and 21). The solution feed may be driven using a syringe pump (Razel Scientific Instruments) and an electrospinning voltage ranging from 15 to 30 kV can be applied between the needle and the collector (aluminium foil) by the use of a Spellman SL10 power supply. The positive electrode of a high voltage power supply is connected to a metal capillary by copper wires. The distance needle tip - collector was 15 cm, and the flow rate of the solution may be 0.75 ml/h. All electrospinning experiments may be performed at room temperature. The nanofibrous nonwoven mats can be collected on the surface of aluminum foil. The as-spun chitosan membranes prepared with the electrospinning method mentioned above are highly positively charged, thus they dissolve in acids and exhibit a low stability in neutral or weak alkaline aqueous media. As far as these membranes are foreseen in tissue engineering, their stability in cell culture and physiological media is required, which explains why many researchers have tried to address this problem either by chemical cross-linking or by neutralization of the salt residues formed when the chitosan is dissolved in acidic solutions. In the present approach, chitosan, as apparent from the appended examples, water-insoluble electrospun membranes were prepared using improved conditions for electrospinning and stabilization process, i.e. advantageously without the use of chemical cross-linkers or chlorine-containing organic solvents. The process for the preparation of a layered electrospun chitosan scaffold may be performed by applying an electrospinning voltage between 15 and 30 kV, or the electrospinning voltage is 15, 17, 20, 23, 25, 27 or 29 kV.
During electrospinning, the 3D structure is provided by the progressive accumulation of the electrospun nanofiber. For instance, the use of a movable collector could allow the guidance of the electrospun nanofibers and the development of specific fiber networks. The more it is electrospun (several nanofibers at the same time and/or a longer electrospinning process) the thicker the nanofiber layer will be. There is no additional crosslink since the overall structure is stabilized by further wetting for neutralization. The diameter of the nanofiber of the electrospun chitosan nanofiber scaffold membrane may range from 50 to 300nm (510⁻⁸ to 310⁻⁷ m). The layers may be stabilized by a subsequent treatment of said chitosan scaffold with an alcohol, such as ethanol, and a basic chemical, such as NaOH, and rinsing with water. Hence, for improving the water stability of the electrospun material, the layered scaffold may be neutralized in a preferred embodiment by a base, preferably by NaOH, and for example NaOH 1M solution. Subsequently, the membranes may extensively be rinsed preferably with distilled water and dried preferably under the vacuum. Both infrared spectroscopy and thermal analysis (DSC) evidence the complete removal of the PEO from the fibers during the stabilization treatment. The low PEO content (10%) of the starting electrospinning solution also favours the stability of the resulting chitosan fibers after stabilization. As evidenced by the SEM analysis (see below), neutralization by NaOH and extensive washing in water surprisingly results in the long-term stability of the chitosan nanofibers in water and phosphate buffers. Stabilized membranes can be stored in distilled water for months without any apparent change of the morphology.
In a preferred embodiment, the layered chitosan scaffold of the present invention is a scaffold, wherein the stabilized chitosan scaffold is freeze-dried resulting in porous layers. Thus, the layered chitosan scaffold is swollen in water and then freeze-dried by first reducing the temperature to -20°C (in a cool room) or at -55°C (by dry ice) until complete solidification of the liquid (water), then the volatiles are removed by sublimation in a lyophilizator under reduced pressure (below 0.1mmHg). In this context, as used herein, the term "freeze-drying" may relate to a rapid and easy way to provide electrospun membranes and/or support layers containing pores of desired size. By controlling the membrane swelling degree, the cooling rate and the final temperature (-20°C or -55°C), the pores sizes can be adjusted. This novel process has never been used to control the porosity in electrospun membranes. Thus, in a preferred embodiment, after stabilisation treatment, dressing (nanofibers and sponge) may be freeze-dried. During the freezing process growing ice crystals push back and concentrate the fibers into the inter-crystals space. After complete solidification, the frozen dressing may be lyophilised (to sublimation of the ice under vacuum), resulting in a fibers mat with pores of the size and shape of the initial ice crystals. As described in detail below, depending on the freezing conditions the size of the ice crystals can be modulated, regulating accordingly the size of the resulting pores in the nanofiber layer of the mat. This treatment will improve the rapid colonization of the dressing (when desired) by increased cells penetration.

Accordingly, in a preferred embodiment, the present invention relates to a scaffold, wherein porosity is controlled by freeze-drying which comprises the step of (a) freezing the chitosan scaffold swollen in water; (b) lyophilising the frozen chitosan scaffold after solidification; and (c) collecting the resulting porous chitosan scaffold. In this context, the term "freezing" may relate to the phenomenon that occurs when the working temperature is below the freezing point of a liquid and thus causes its crystallization. In addition, in this context, the term "drying" may relate to the phenomenon that occurs when a liquid is removed by sublimation such as in a lyophilisation process.

The process may comprise the freeze-drying of the whole scaffold after the chitosan nanofibres have been deposited, such as by electrospinning, onto the porous layer.
In accordance with the above, and as shown in the appended examples, the pore size that can be created in the electrospun membrane is 10 microns on an average after freeze-drying. In contrast, the pore size obtained by regular electrospinning without subsequent freeze-drying varies from 100 nm to 1 micron (10⁻⁷ to 10⁻⁶ m). In the porous support layer, the pore size may be much higher. The lower limit of the pore size to allow better cell invasion is around the average diameter of a cell (i.e. 1 micron or 10⁻⁶ m). As regards the higher limit of the pore size, there is no clear limit except for the fact that the cells must stay close to the electrospun material, i.e. that too many pores of too big size are not expected to favour interaction of the material with the cells. Consequently, in a preferred embodiment of the invention, the size of the pores of the porous chitosan scaffold is below 500 microns (5 10⁻⁴ m) for the support layer (chitosan sponge). In a preferred embodiment, said pores range from 50 to 500 microns (5 10⁻⁵ to 5 10⁻⁴ m). In a more preferred embodiment, said pores range from 50 to 400, from 50 to 300 microns (10⁻⁶m), from 50 to 200 microns(10⁻⁶m) or from 50 to 100 microns (10⁻⁶ m). For the chitosan nanofiber membrane the size of the pores are below 100 microns. In a preferred embodiment, the pores of the chitosan nanofiber membrane range from 1 to 100 microns (10⁻⁶m), from 1 to 80 microns (10⁻⁶m), from 1 to 50 microns (10⁻⁶m), or from 1 to 30 microns (10⁻⁶m). It was surprisingly found in the context of the present invention that the first layer and the support layer partially fuse when chitosan solution is electrospun onto the porous chitosan scaffold support layer. This is because, when chitosan solution is electrospun onto a chitosan supporting sponge, traces of solvent that are still in or on the nanofibers are abundant enough to partially swell the chitosan sponge, resulting in a partial fusion between the nanofibers and the underlying chitosan material. Hence, in a preferred embodiment, the present invention relates to a scaffold, wherein the chitosan nanofiber membrane and the chitosan support layer are partially fused when chitosan solution is electrospun onto the porous chitosan scaffold support layer.
In a further aspect of the invention, the chitosan of at least one layer of the layered chitosan scaffold may partially or totally be reacetylated into chitin.

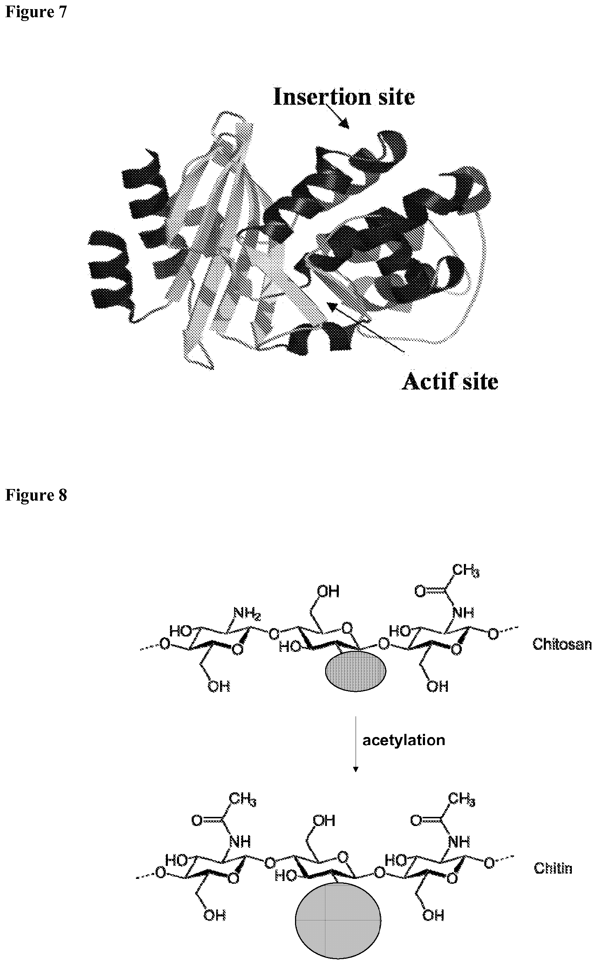

The goal of the convertion of electrospun chitosan nanofibers to chitin nanofibers is to permit the following:
- To maintain both the fibrillar structure of the nanofibers and the morphology of the biopolymer
- To restore the sensitivity to human glycosylhydrolases
- To control the reacetylation degree ,also called degree of acetylation
- To combine chemical, biophysical and biological properties of chitosan and chitin in the same biopolymer.
The chemical conversion of chitosan consists of the substitution of the C2 amine (-NH2) group by an acetamide group (-NHCOCH3) with acetate anhydride (Figure 8).

As explained in detail below, the chitosan of the layered chitosan scaffold may partially or totally be reacetylated into chitin by a chemical process which permits the reacetylation of electrospun chitosan nanofibers and without altering the fibrillary structure of the biomaterial. As electrospun chitosan nanofibers are poorly biodegradable, the reacetylation permits to restore sensitivity to human glycosyl hydrolases such as the human lysozyme and the human macrophage chitotriosidase. The term "partially or totally reacetylated" may relate to a layered chitosan scaffold, in which the ratio of chitosan/chitin is altered or modified compared to an unmodified scaffold that consists of 100% chitosan. In sum, the percentage of chitosan + the percentage of chitin is 100%. Hence, the percentage of chitosan in said scaffold may be between 0% and 100%. Accordingly, the percentage of chitin in said scaffold may be between 100% and 0%. In a preferred embodiment, the percentage of chitosan is 1%, 2%, 3%, 5%, 7%, 10%, 15%, 20%, 25%, 30%, 35%, 40%, 45%, 50%, 55%, 60%, 65%, 70%, 75%, 80%, 85%, 90%, 95%, 98% or 99%. Correspondingly, the percentage of chitin in said scaffold is 99%, 98%, 97%, 95%, 93%, 90%, 85%, 80%, 75%, 70%, 65%, 60%, 55%, 45%, 40%, 35%, 30%, 25%, 20%, 15%, 10%, 5%, 2% or 1%. The ratio of chitosan/chitin can be modified by controlling the chemical conversion process, i.e. by modulating the acetylation degree of chitosan. Hence, by controlling the acetylation degree of chitosan, it is possible to develop biopolymers that, in context of the present application, are more or less biodegradable. In addition, modulation of the acetylation degree permits to combine the specific properties of chitosan and chitin in the same biopolymer. For instance, these properties are the tensile strength, elasticity of the nanofibers, air permeation, hydrophobicity. Furthermore, it has to be noted that the reacetylation of the present invention surprisingly does not alter the fibrillar structure of the nanofibers and the morphology of the electrospun chitosan biopolymer (see below). In summary, the chitosan scaffold may partially or totally be reacetylated into chitin by a chemical process and said chemical conversion can be controlled, the acetylation rate of chitosan can be modulated. This feature is important because it allows restoring the susceptibility to human glycosylhydrolases and it permits to develop biopolymers that, in the context of the application, are more or less biodegradable. In addition, modulation of the reacetylation rate permits to combine the specific properties of chitosan and chitin in the same biopolymer. Degree of acetylation (DDA) of chitosan, solvent used to dilute acetate anhydride, dilution range of acetate anhydride, thickness and surface of the biomaterial, presence or absence of an additional layer such as a sponge, diameter of the electrospun nanofibers, porosity of the biomaterial, incubation times, methodology to wash and to air-dry the reacetylated biopolymer, and methodology used to produce and to stabilize the biopolymer are all key parameters that modulate the conversion ratio of chitosan to chitin. The fact that the nanofibrillar structure of the electrospun chitosan nanofibers is maintained or not after reacetylation, also depends of these parameters.
Accordingly, in a preferred embodiment, the present invention relates to a scaffold, wherein chitosan nanofibres has a degree of deacetylation comprised between 50 and 100 %.
As surprisingly found in the present application and as illustrated in the appended examples, the reacetylation process of the conversion of chitosan to chitin of the present invention results in nanofibers without altering its morphology. This is because the chemical reaction of reacetylation of the present invention does not require chitosan dissolution and does not imply multifunctional reactant that would lead to cross-linking. Hence, the reacetylation process of the present invention does not destroy the nanofiber morphology. This process gives the opportunity to obtain electrospun chitin nanofibres (50 to 300 nm)(510⁻⁸ to 310⁻⁷ m). In view of the prior art, this was thought to be impossible because of the insolubility related to chitin biomaterial itself. Furthermore, due to the different characteristics (solubility, degradability, biocompatibility, ...) of chitin and chitosan, the properties and functions of chitosan/chitin-based biomaterial may be easily adjusted.

Accordingly, in a preferred embodiment, the present invention relates to a scaffold wherein the reacetylation of chitosan into chitin is partially or totally adjusted to modulate properties such as the biodegradation level. In context of the present application, the term "biodegradation level" refers to the speed at which the molecular weight of chitosane is significantly reduced by enzymes so that the 3D morphology is affected. Therefore, in a further preferred aspect, the present invention relates to a scaffold, wherein the chemical structure and composition of chitosan is unaltered by cross-linking or other chemical reactions. In the context of the present invention, the term "chemical reactions" is well known in the art and refers to any chemical modification by covalent bonds formation or rupture. The term "unaltered" means that chitosan has a degree of deacetylation comprised between 0 (chitin) and 100 % and does not contains any other chemical modification. In another embodiment, unaltered may mean also that the chitosan scaffold retains its biological properties. Consequently, the present invention relates to a scaffold wherein the chemical structure and composition of chitosan are unaltered, i.e. the chemical composition of the chitosan molecules is not modified since no cross-linking with chemicals or cross-linkers is performed and said chitosan molecules are not treated with chemical compounds or chemicals. Therefore, in a preferred embodiment, the biological properties of the chitosan molecules is not affected.

In a preferred embodiment, the scaffold of the present invention is a scaffold, wherein the porous and nanofiber morphologies are both preserved during immersion in aqueous media (at least neutral and basic) and other solvents. In the context of the present application, the term "preservation of the morphology" is well known in the art and refers to pores size as well as to the diameter and length of the fibers that are unalterred by the treatment as described above. In this context, the person skilled in the art is readily in a position to determine whether the morphology is preserved by environmental scanning electron microscope observations. Furthermore, in the context of the present invention, the neutral and basic aqueous media can, e.g. be phosphate buffers or NaOH solutions well known to the skilled person. As regards the term solvents, this term is well known in the art and the scaffold of the present invention can be immersed in other solvents, like alcohols or ethers, wherein the porous and nanofiber morphologies are both preserved during said immersion.

In accordance with the above and as shown in the appended examples, the present invention relates to a scaffold, wherein active agents or additives are selected from the group consisting of proteins, enzymes, complex biological molecules, DNA molecules, RNA molecules, ions, molecules preventing denaturation, misfolding or aggregation of proteins, molecules having antibacterial, antifungal or antiviral properties are incorporated in the nanofiber layer.

In the following, the process for reacetylation is described in detail. In a further aspect of the present invention, there is provided a process for the reacetylation of electrospun chitosan nanofibers, wherein the nanofibers are first stabilized by neutralization with a base, for example NaOH, rinsed for example with water, dried for example under the vacuum and next treated with an anhydride acetate solution, rinsed with water and dried under the vaccum. In a preferred embodiment, the solvent used to dilute acetate anhydride is an organic solvent and more preferably is methanol. In a preferred embodiment, the range of acetate anhydride dilutions into methanol varies between 6.5% and 0.75% and more preferably is 3.12%. In a preferred embodiment, the incubation times varies between a brief soaking and two hours.

The present invention relates to a process for the preparation of a layered chitosan scaffold of the present invention, wherein the scaffold of the invention is prepared by (a) preparing a sponge ; (b) depositing chitosan nanofibers onto said chitosan sponge ; and (c) freeze-drying of the product resulting from step (b) for obtaining the layered chitosan scaffold.

Consequently, the present invention provides in a further preferred embodiment for a process of a layered chitosan scaffold, wherein the scaffold of the invention is prepared depositing chitosan nanofibers onto said porous chitosan support layer. As explained in detail below, in a preferred embodiment, the term "depositing chitosan nanofibers onto a chitosan sponge" relates an electrospinning process, wherein said electrospinning process may, in yet another preferred embodiment, be the electrospinning a solution of chitosan with a polymer improving its electrospinning ability such as PEO, on an electrospinning collector covered by a chitosan sponge. However, the term "depositing chitosan nanofibers onto a chitosan sponge" is not only bound to electrospinning. Therefore, the depositing of chitosan and/or the nanofibres deposited onto a chitosan sponge may also be performed by other methods known in the art.

Consequently, in accordance with the above and in yet another further preferred embodiment, the present invention relates to a process for the preparation of a layered chitosan scaffold comprising the steps of: (a) covering an electrospinning collector by a chitosan sponge; (b) electrospinning on said collector a solution of chitosan with a polymer improving its electrospinning ability such as PEO ; (c) wetting the layered chitosan scaffold to remove the polymer and neutralizing the scaffold for stabilization purposes (i.e. preventing its solubilisation in neutral aqueous solutions); and (d) freeze-drying of the water-swollen stabilized scaffold for the control of the porosity.

In accordance with the above, the entire chitosan scaffold is partially reacetylated into chitin when it is soaked in methanol containing acetate anhydride. As exemplified in the appended examples, it is surprisingly demonstrated that the conversion of chitosan into chitin is performed without destroying the nanofibers.

Accordingly, in a further preferred embodiment, the present invention relates to a process, wherein the chitosan is partially or totally reacetylated, as already in detail explained above, in order to modulate properties such as the biodegradation level.

In a further aspect, the present invention relates to a process for the preparation of a layered chitosan scaffold, wherein in the electrospinning process active agents and/or additives are co-electrospun with chitosan. This process is basically performed in accordance to the electrospinning process as described above. However, additives are added in the chitosan/PEO mixture and electrospinning procedures are subsequently used as described above. As illustrated in the appended examples, it was found that active agents and/or additives, e.g. proteins, maintain their activity after electrospinning.

In a further preferred embodiment, active agents and/or additives which may be electrospun with chitosan may be selected from the group consisting of proteins, enzymes, complex biological molecules, DNA molecules, RNA molecules, ions, or any chemical molecules having the ability to maintain and to preserve the biological activity of the proteins during the electrospinning or the storage of the electrospun biomaterial. Such molecules would act as chaperone molecules capable of stabilizing protein structure during water deficit, salt and temperature stresses. These chemical molecules could be, among others, sugars, polyols, amino acids, and methyl-amines. Additives could be also molecules having antimicrobial properties, meaning that such molecule has a destructive or an inhibiting effect on bacteria, virus or fungi.

In a preferred embodiment, the present invention also relates to a process, wherein the layered scaffold is produced by freeze-drying of the stabilized chitosan scaffold. In a preferred embodiment, the freeze-drying comprises the step of (a) freezing the stabilized chitosan scaffold obtained by the process as described above; (b) lyophilising the frozen chitosan scaffold after solidification; and (c) collecting the resulting porous chitosan scaffold. In this context, as used herein and already explained in detail above, the term "freeze-dryed" may relate to a rapid and easy way to provide electrospun membranes and/or support layers containing pores of desired size. This process has never been used for the creation of pores in electrospun membranes. Thus, in a preferred embodiment, after stabilisation treatment, dressing (nanofibers and sponge) may be washed and frozen. During the freezing process growing ice crystals push back and concentrate the fibers into the inter-crystals space. After complete solidification, the frozen dressing may be lyophilised (to sublimation of the ice under vacuum), resulting in a fibers mat with pores of the size and shape of the initial ice crystals. In a preferred embodiment, as described in detail below, depending on the freezing conditions the size of the ice crystals can be modulated, regulating accordingly the size of the resulting pores in the nanofiber layer of the mat. This treatment will improve the rapid colonization of the dressing (when desired) and increase its permeability to air, oxygen, water vapour and liquid water.

In accordance with the above, and as shown in the appended examples, the pore size that can be created in the electrospun membrane is 10 microns (or 10⁻⁵ m) on an average after freeze-drying. In contrast, the pore size obtained by regular electrospinning without subsequent freeze-drying varies from 100 nm to 1 micron (10⁻⁷ to 10⁻⁶ m). In the support layer made of sponge, the pore size may be much higher. In general, the lower limit of the pore size to allow better cell invasion is around the average diameter of a cell (i.e. 1 micron or 10⁻⁶ m). As regards the higher limit of the pore size, there is no clear limit except for the fact that the cells must stay close to the electrospun material, i.e. that too many pores of too big size are not expected to favour interaction of the material with the cells. Consequently, in a preferred embodiment of the invention, the pores of the porous chitosan and of the chitosan nanofiber membrane have a size as already described above.

Advantageously, in a preferred embodiment, the size of the pores of the support layer (preferably a chitosan sponge) and of the chitosan nanofiber membrane is controlled by modifying the freezing conditions of the freeze-drying. As exemplified in the appended examples, this is an easy and not harmful way to form pores of various and tunable size. This may be important for facilitating cell invasion in vitro (e.g., for tissue engineering) and in vivo (e.g., for progressively degradable wound dressings or implants). In preferred embodiments, the pore size can be controlled and modified by the following parameters: water content, cooling rate and freezing temperature.

In yet another preferred embodiment, the present invention relates to a process of preparing a layered electrospun chitosan scaffold wherein the first layer is electrospun on the support layer, wherein the support layer and the first layer partially fuse by electrospinning of the chitosan solution onto the porous chitosan scaffold support layer. This is because, it was surprisingly found in the context of the present invention that the first layer and the support layer partially fuse when chitosan solution is electrospun onto the porous chitosan support layer because, when chitosan solution is electrospun onto a chitosan supporting sponge, traces of solvent that are still in or on the nanofibers are abundant enough to locally swell the chitosan sponge, resulting in a partial fusion between the nanofibers and the underlying chitosan material.

In another preferred embodiment, the present invention relates to a process of preparing a layered electrospun chitosan scaffold, wherein the chitosan in at least one layer is partially or totally reacetylated into a chitin without modifying its structure.

As can be derived from the appended examples, only very specific concentrations of acetate anhydride in methanol allow the reacetylation of chitosan into chitin without affecting the structure of the scaffold. Yet, without being bound to these concentrations, in a preferred embodiment, the concentration of acetate anhydride is 3.12 % in methanol, although other concentrations of acetylating agents in other types of alcohol or solvent may be used. Consequently, in another preferred embodiment, the reacetylation of chitosan into chitin is performed by incubating said chitosan nanofiber scaffold with acetate anhydride and in any other organic solvents known to the person skilled in the art. In this context, it is not only envisaged to use methanol as an organic solvent. Also other organic solvents and/or alcohols can be used. Other organic solvents and/or alcohols are known to the person skilled in the art and the person skilled in the art is in a position to chose appropriate other organic solvents and/or alcohols which may comprise, inter alia, e.g.. As already mentioned above, it is not only envisaged to use acetate anhydride as an acetylating agent. Hence, in another preferred embodiment, the reacetylation of chitosan into chitin is performed by incubating said chitosan nanofiber scaffold with any other acetylating agent known to the person skilled in the art. Other acetylating agents are known to the person skilled in the art and the person skilled in the art is in a position to choose appropriate other acetylating agents which may comprise, inter alia, e.g.. As regards the concentration of acetate anhydride it is not only envisaged to use a concentration of 3.12%. Consequently, the concentration acetate anhydride may range from 6.5 to 0.75 %. The concentration of the acetate anhydride may be 0.75%, 1%, 2%, 3%, 4%, 5%, 6%, 6.25% and 6,5%. The concentration of the acetate anhydride may be 3,12%. It may be also envisaged that the beforementioned concentrations for acetate anhydride also apply, mutatis mutandis, for the appropriately chosen other acetylating agent(s) mentioned above.
The person skilled in the art, based on his general knowledge, will realise that incubation times strongly depend upon the thickness of the chitosan scaffold (nanometric or micrometric structures), the type of chitosan (highly deacetylated or not) and the level of reacetylation that is required/desired.
In accordance with the above, the chitosan in the entire chitosan scaffold is partially reacetylated into chitin when it is soaked in methanol containing acetate anhydride. As exemplified in the appended examples, it is surprisingly demonstrated that the conversion of chitosan into chitin is performed without destroying the nanofibers.

As already explained above, it was surprisingly found in the context of the present application and as illustrated in the appended examples, that the reacetylation process of the conversion of chitosan to chitin of the present invention results in nanofibers without altering its structure. This is because, the chemical reaction of reacetylation of the present invention does not add chemical groups initially absent in chitosan. Hence, the reacetylation process of the present invention does not destroy the nanofiber structure. This process gives the opportunity to obtain electrospun chitin fibers. In view of the prior art, this was thought to be impossible because of the insolubility related to chitin. Furthermore, due to the different characteristics (solubility, degradability, biocompatibility) of chitin and chitosan, the properties and functions of chitosan/chitin-based biomaterial may be easily adjusted. As explained in detail below, the layered chitosan scaffold may partially or totally be reacetylated into chitin by a chemical process which permits the reacetylation of electrospun chitosan nanofibers and without altering the fibrillary structure of the biomaterial. In the context of the process of preparing a layered electrospun chitosan scaffold of the invention, the term "partially or totally reacetylated" may relate to a layered chitosan scaffold, in which the ratio of chitosan/chitin is altered or modified compared to an unmodified scaffold that consists of 100% chitosan. In sum, the percentage of chitosan + the percentage of chitin is 100%. Hence, the percentage of chitosan in said scaffold and the percentage of chitin in said scaffold may have percentages as already described above. In the context of the process of preparing a layered electrospun chitosan scaffold, it is understood that the ratio of chitosan/chitin can be modified by controlling the chemical conversion process, i.e. by modulating the acetylation rate of chitosan. Hence, by controlling the reacetylation rate of chitosan, it is possible to develop biopolymers that, in the context of the application, are more or less biodegradable. In addition, modulation of the reacetylation rate permits to combine the specific properties of chitosan and chitin in the same biopolymer. For instance, these properties are the tensile strength, elasticity of the nanofibers, air permeation, hydrophobicity. It does not alter the fibrillar structure of the nanofibers and the morphology of the electrospun chitosan biopolymer. In summary, the chitosan scaffold may partially or totally be reacetylated into chitin by a chemical process and said chemical conversion can be controlled, the acetylation rate of chitosan can be modulated. This feature is important because it allows restoring the susceptibility to human glycosylhydrolases and it permits to develop biopolymers that, in function of the application, are more or less biodegradable. In addition, modulation of the reacetylation rate permits to combine the specific properties of chitosan and chitin in the same biopolymer.
In a preferred embodiment, the present invention relates to a process of preparing a layered electrospun chitosan scaffold, wherein the chitosan in the chitosan nanofiber scaffold is partially or totally reacetylated by incubating said chitosan nanofiber scaffold with acetate anhydride and organic solvents. The concentration of acetate anhydride may range from 6.5 to 0.75 % or may be 3.12 %.

In yet another preferred embodiment, the present invention relates to a process of preparing a layered electrospun chitosan scaffold, wherein the chitosan in the chitosan nanofiber scaffold is partially or totally reacetylated by incubating said chitosan nanofiber scaffold with acetate anhydride and organic solvent, wherein the the organic solvent is selected from the group consisting of ethanol, methanol, acetone, dimethyl formamide, heptane and tetrahydrofurane. In a more preferred embodiment, the organic solvent is methanol.
As detailed explained above, the present invention relates to a process of preparing a layered electrospun chitosan scaffold, wherein the reacetylation rate of chitosan is controlled/modulated. In addition, in yet another preferred embodiment, the rate is controlled to obtain a desired chitosan/chitin ratio that has a desired biodegradability. Hence, by controlling the reacetylation rate of chitosan, it is possible to develop biopolymers that, in the context of the present application, are more or less biodegradable. In addition, modulation of the reacetylation rate permits to combine the specific properties of chitosan and chitin in the same biopolymer as already described above.

Finally, in a preferred embodiment, the present invention relates to the layered electrospun chitosan scaffold of the present invention or the layered electrospun chitosan scaffold produced by the process of the present invention for use as a wound dressing, in regenerative medicine that involves multidisciplinary fields such as biology, medicine, and engineering, and that consists to restore, to maintaining, or to enhance tissue and organ function. In addition to having a therapeutic application, where the tissue is either grown in a patient or outside the patient and transplanted, tissue engineering can have diagnostic, prognostic or pharmaceutical applications where the tissue is made in vitro and used for testing drug metabolism and uptake, toxicity, and pathogenicity. The rationale for tissue engineering/regenerative medicine for either therapeutic or diagnostic applications is the ability to exploit living cells in a variety of ways.

Tissue engineering research includes the following areas:
1) Biomaterials: including novel biomaterials that are designed to direct the organization, growth, and differentiation of cells in the process of forming functional tissue by providing both physical and chemical cues.
2) Cells: including enabling methodologies for the proliferation and differentiation of cells, acquiring the appropriate source of cells such as autologous cells, allogeneic cells, xenogeneic cells, stem cells, genetically engineered cells, and immunological manipulation.
3) Biomolecules: including angiogenic factors, growth factors, differentiation factors, adhesion factors, antimicrobial factors...
4) Engineering Design Aspects: including 2-d cell expansion, 3-d tissue growth, bioreactors, vascularization, cell and tissue storage and shipping (biological packaging).
5) Biomechanical Aspects of Design: including properties of native tissues, identification of minimum properties required of engineered tissues, mechanical signals regulating engineered tissues, and efficacy and safety of engineered tissues

The term biological application may relate to therapeutic application and diagnostic application.

In summary, in the context of the present invention, it was unexpectedly found that electrospinning dramatically improved the properties of chitosan as substrate for growing primary connective tissues cells in culture. Moreover, electrospinning also dramatically improved the biocompatibility of chitosan in vivo as compared to film and sponges for examples, which was never shown or suggested before. This of course opens routes to clinical applications. Accordingly, the present invention advantageously provides:
1. Electrospinning of chitosan onto chitosan sponges:
   - it allows easy manipulation of electrospun material
   - it improves its mechanical resistance
   - the sponge can absorb exudates better than electrospun material
   - there is partial fusion between nanofibers and sponges, which could not be easily obtain with system using different macromolecules for forming sponges and nanofibers
   - the partial fusion allows firms adhesion between the two "layers" but would allow also to remove the sponge layer without removing the electrospun layer. This is of crucial importance when the "inner" electrospun layer in contact with the wound is invaded with host cells during the healing process. Indeed, its removal would alter the healing process. Instead, this electrospun layer is biocompatible and will be progressively degraded and replaced by a new extracellular matrix deposited by invading and not hyperactivated cells (as exemplified in the appended examples during in vivo experiments).
2. Modifications and stabilization of the electrospun layer
   - Stabilization of the biomaterial by neutralization without cross-linking. This improvement over the previous state of the art allows to work without toxic chemicals and insure long-term stability of the morphology even upon immersion in liquids such physiological water. Preserve the initial chemical structure and composition of the chitosan, i.e. chemical properties.
   - Freeze-drying to confer various porosity to the electrospun layer. This is an easy and not harmful way (that was never reported before) to form pores of various and tunable size. It may be important for facilitating cell invasion in vitro (for tissue engineering for example) and in vivo (for progressively degradable wound dressings or implants for example).
   - A reacetylation process of chitosan into chitin that preserves the nanofiber morphology is completely new and required a lot of work and inventiveness. This process gives the opportunity to obtain electrospun chitin nanofibers, which was thought to be impossible because of problems related to insolubility of chitin. Furthermore, due to the different characteristics (i.e., e.g, solubility, degradability, biocompatibility) of chitin and chitosan, it will allow to "tailor" more easily the properties and function of chitin/chitosan biomaterial.

The following Figures show and illustrate the present invention:

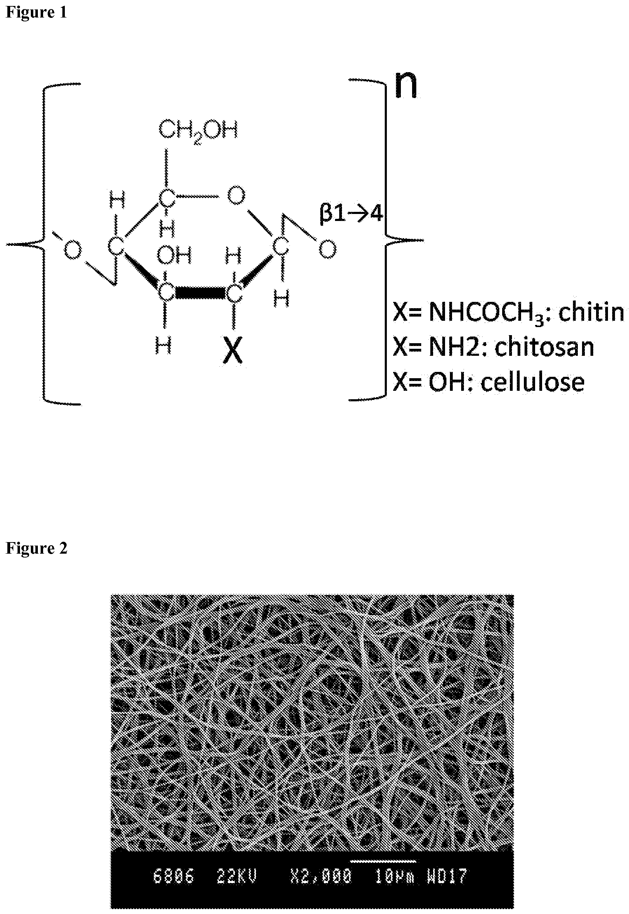

**Figure 1****. Schematic representation of chitin, chitosan and cellulose.**
**Figure 2****: Scanning electron microscopy showing the nanofiber structure of chitosan electrospun membranes.**

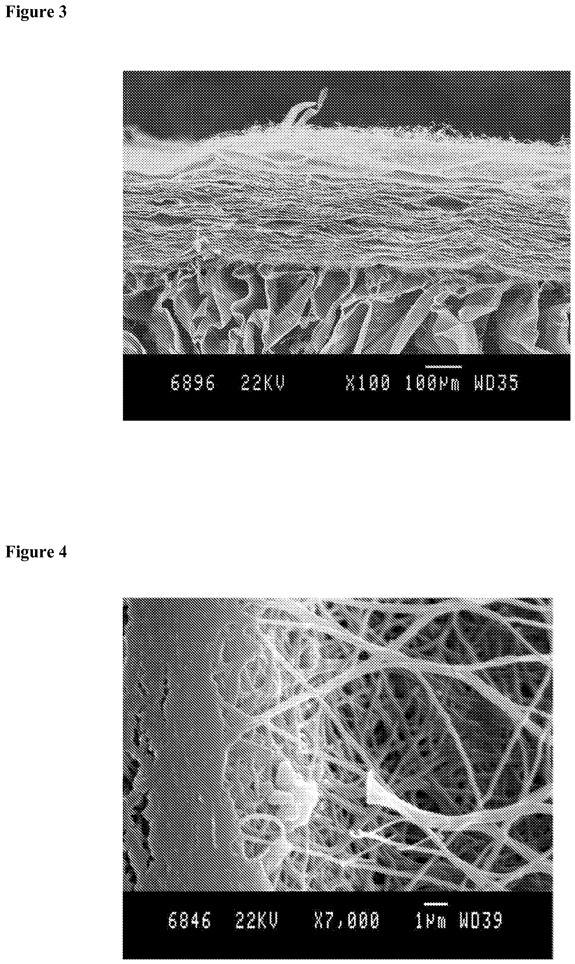

**Figure 3****. Cross section of a multilayer dressing visualized by scanning electron microscopy.** Nanofibers of chitosan are visible at the top of the chitosan sponge
**Figure 4****. Cross section of the junction between the first (electrospun nanofibers) and the second (chitosan sponge) layers of a prototype dressing.** Partial fusion between the two layers is observed (by scanning electron microscopy).

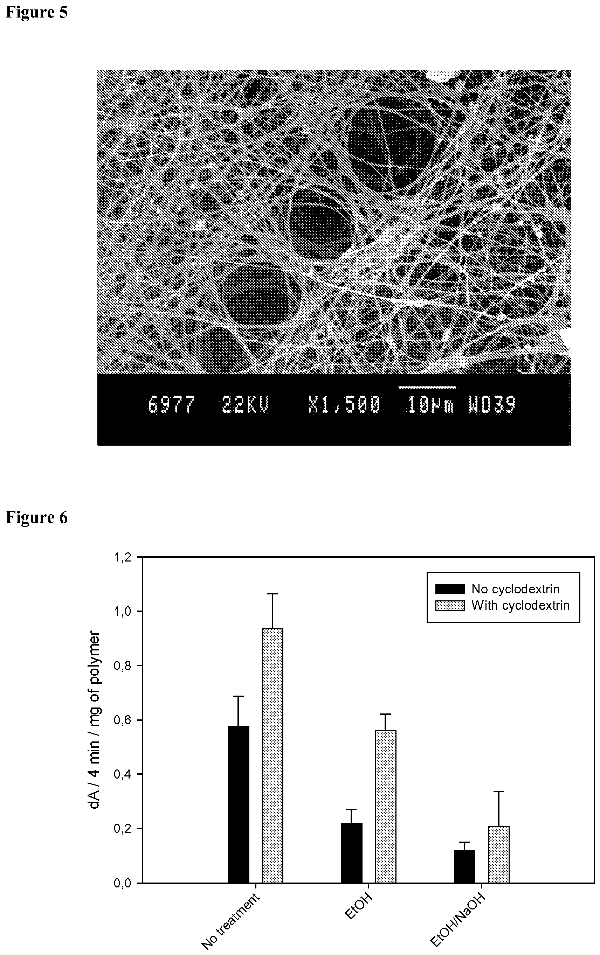

**Figure 5****. Observation by scanning electron microscopy of pores formed in the nanofiber compartment of a two layers dressing by freeze-drying.**
**Figure 6****: Immobilized β-lactamase activity per mg of dry polymer.** Assays were performed by incubating each polymer with 1 ml of 100 µM nitrocefin during 4 minutes. Absorbance of the supernatant was then measured at 482 nm. Electrospun membranes were either not stabilized (No treatment), submitted to ethanol bath (EtOH) or fully stabilized according to our new protocol (EtOH/NaOH).
**Figure 7****. Globular 3D-structure of the *Bacillus licheniformis* β-lactamase BlaP.**
**Figure 8****. Chemical conversion of chitosan to chitin.**

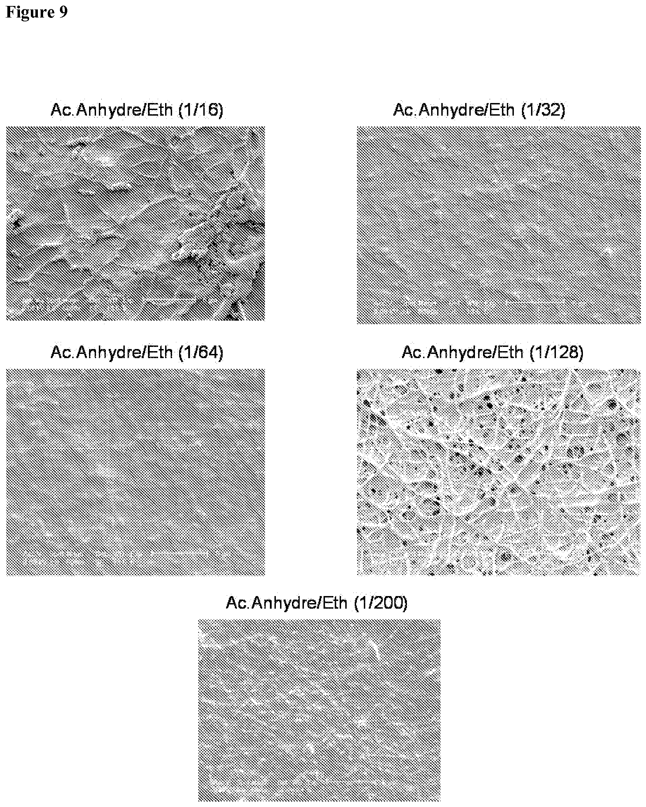

**Figure 9****. Scanning electron microscopy of electrospun chitosan nanofibers re-acetylated with different dilutions of acetate anhydride in ethanol.**

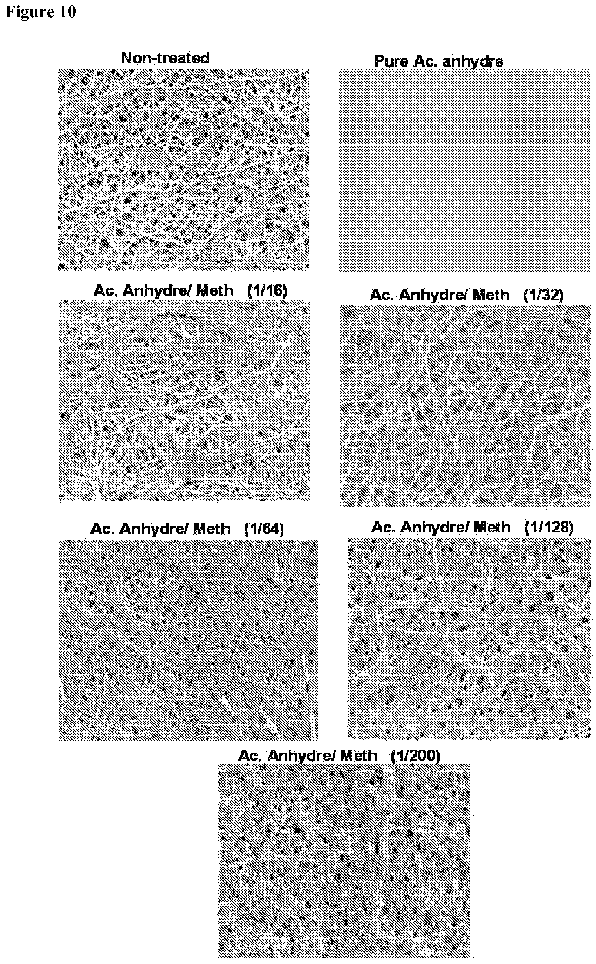

**Figure 10****. Scanning electron microscopy of electrospun chitosan nanofibers re-acetylated with different dilutions of acetate anhydride in methanol.**

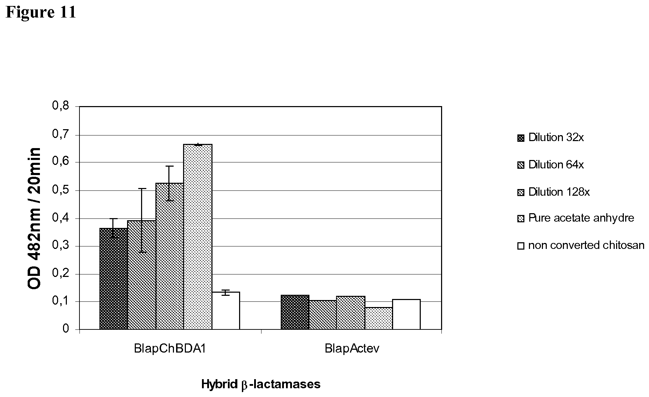

**Figure 11****. Chitin binding assays performed with BlaP ChBDAl and BlaP Actev.** The assays were performed on electrospun nanofibrous membranes of chitosan (square of 1cm²) treated or not with pure acetate anhydride and acetate anhydride diluted (32 to 128X) in methanol. The immobilized β-lactamase was monitored by following the hydrolysis of nitrocefin at 482nm.

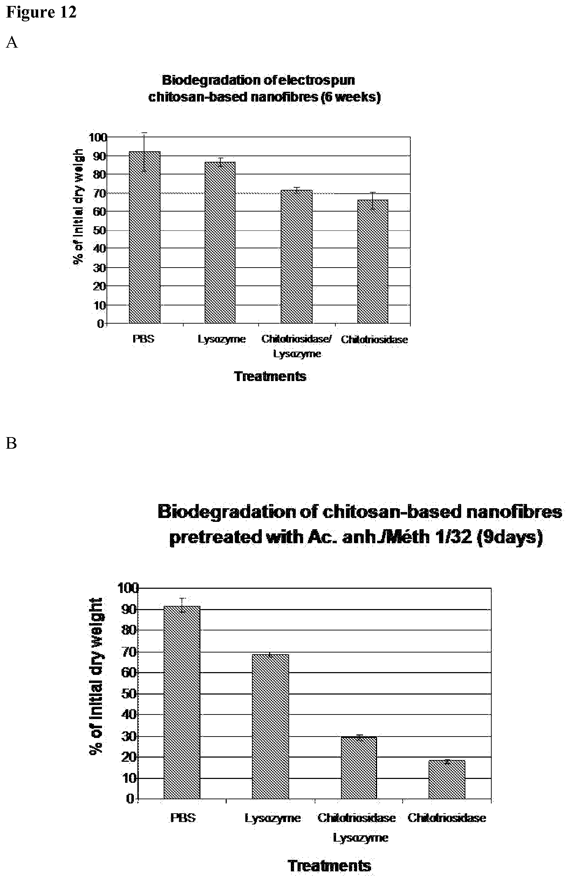

**Figure 12****. Biodegradation assays of electrospun chitosan nanofibers before (A) and after (B) treatment with acetate anhydride diluted in methanol (1/32).**

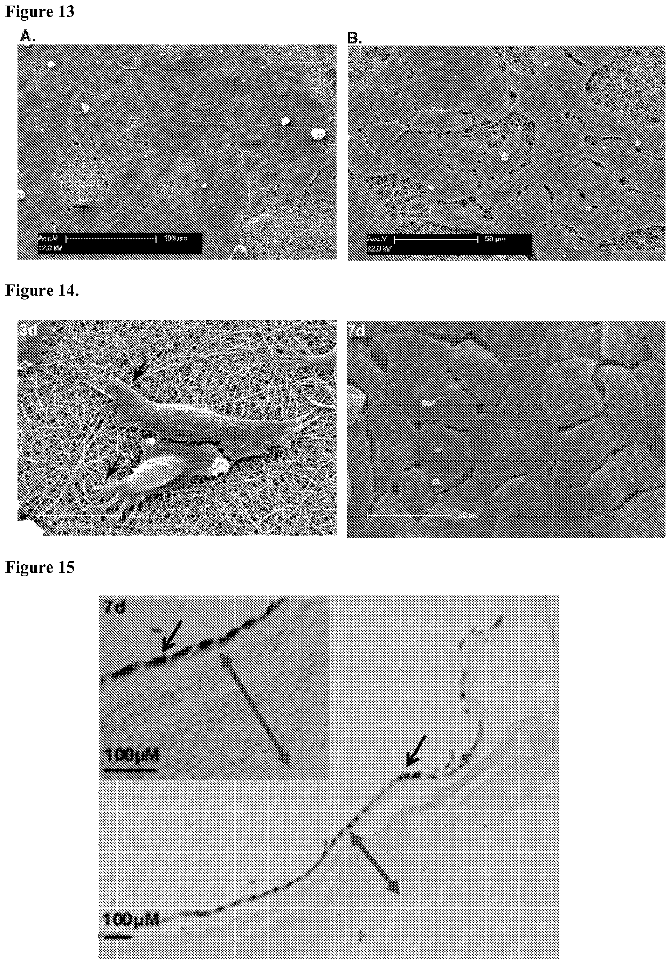

**Figure 13****. Scanning electron microscopy of normal human keratinocytes cultured on electrospun chitosan membranes.** Electrospun chitosan membranes were placed in a 12-well cell culture plates, sterilized and seeded with normal human keratinocytes (30 x 10³cells/cm²). Scanning electron micrographs were taken after 7 days in culture. Original magnification 250 x (A) and 650 x (B).
**Figure 14****. Scanning electron microscopy of normal human keratinocytes cultured on electrospun chitosan membranes for 3 and 7 days.** Scanning electron micrographs of normal epidermal keratinocytes, cultured on electrospun chitosan membranes (30 x 10³cells/cm²), were taken after 3 (A) and 7 days (B). (A) During the first hours after seeding, keratinocytes adhere on the surface of membranes and form lamellipodia and filopodia along the nanofibers (arrows). Few days later (B), the chitosan nanofibers were almost completely covered by cells, and cell to cell contacts are formed.
**Figure 15****. Hematoxylin staining of paraffin embedded chitosan membranes after 7 days of culture with keratinocytes.** Electrospun chitosan membranes (red double heads arrows) covered by keratinocytes (black arrows) were fixed and embedded vertically in paraffin. Then histological staining with hematoxylin was performed on vertical 6µm sections.

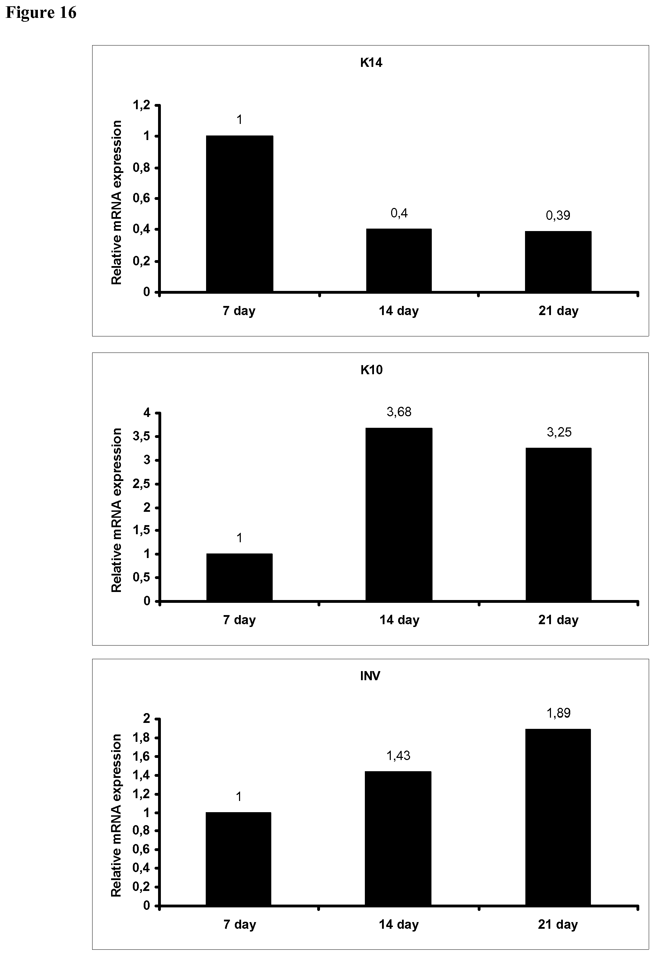

**Figure 16****. Analysis of the differentiation of normal human keratinocytes cultured on electrospun chitosan membranes.** Total RNA was extracted from keratinocytes cultured for various period of time (7, 14 and 21 days) over nanofiber chitosan membranes. After reverse transcription of RNA, the cDNAs of keratin 14 (basal marker), keratin 10 and involucrin (differentiation markers) were amplified with the real-time PCR method and the expression of the epidermal markers of differentiation was analysed after normalization to the expression of the house-keeping gene 36B4.

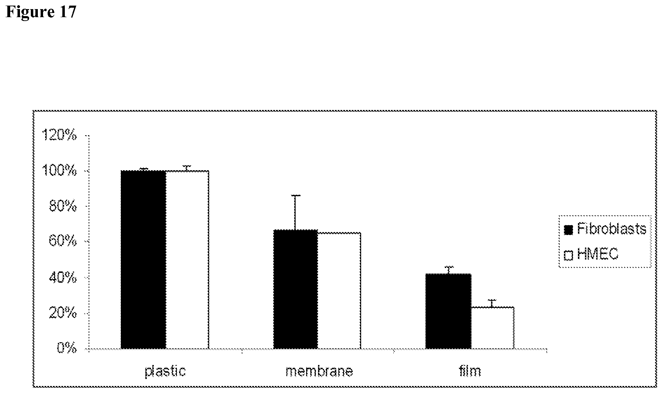

**Figure 17****: Evaluation of cell attachment on different substrates.** Fibroblasts and HMEC were seeded at the same density on plastic, electrospun chitosane membrane and chitosane evaporated film. After one day, a WST-1 assay was performed. Adhesion on culture plastic dishes was considered as 100 %. Adhesion of fibroblasts and endothelial cells (HMEC) was clealy higher on chitosan nanofibers membranes than on evaporated films.

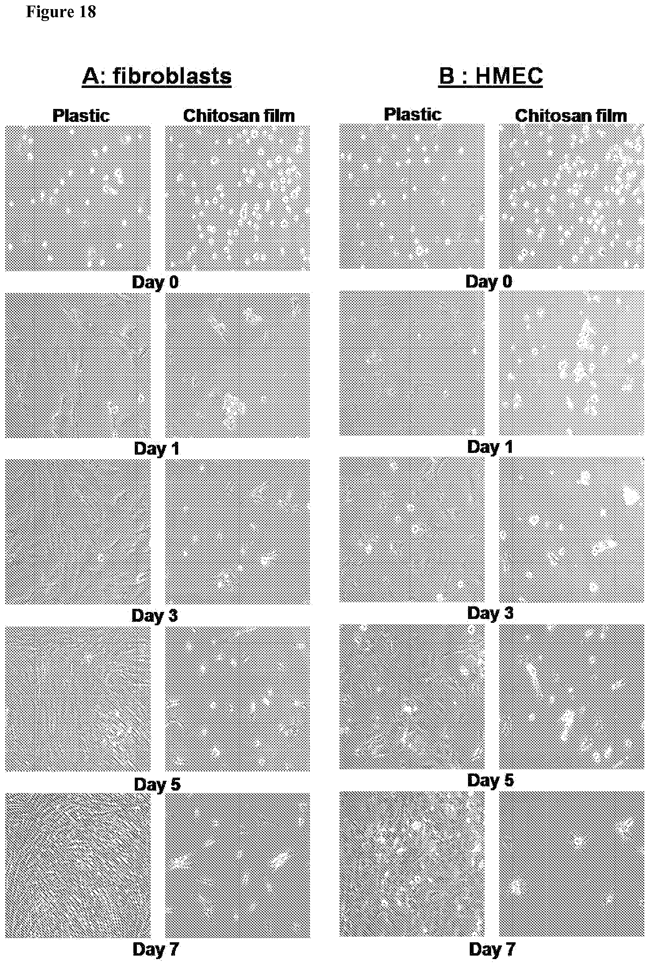

**Figure 18****. Observation of cell attachment, spreading and proliferation by phase-contrast microscopy.** Fibroblasts (A) and HMEC (B) were seeded on plastic and chitosan film for a time-course experiment. No observation could be made with electrospun membranes because of a lack of transparency. Representative pictures were taken at days 0 to 7. Fibroblasts and HMEC attached, spread and proliferated on plastic, forming a complete monolayer at day 7. At the contrary only a limited number of both cell types were able to adhere and partly spread after 3 days. No proliferation was observed, with, at the contrary a cell number being lower at day 7 as compared to day 0 again suggesting cell mortality as shown in Figure 2.

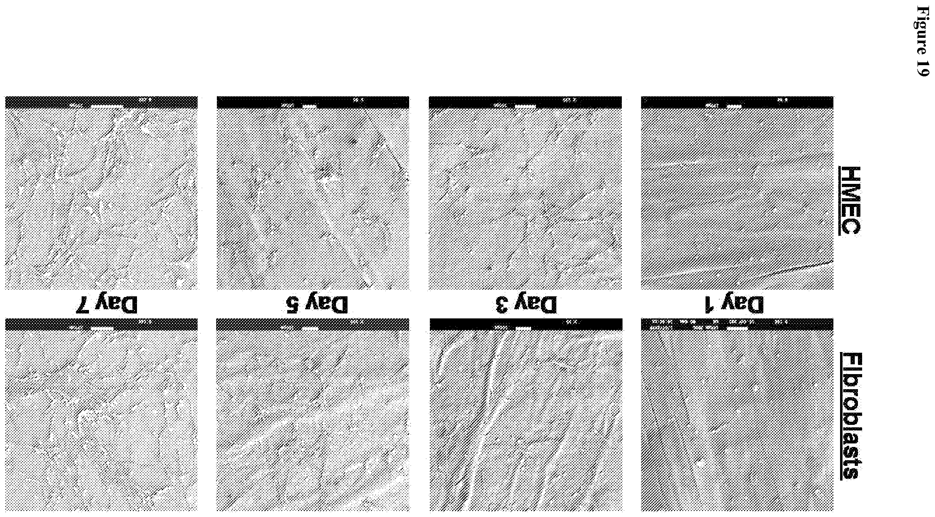

**Figure 19****. Observation by scanning electron microscopy (SEM) of cell attachment, spreading and proliferation.** Fibroblasts and HMEC were seeded on membranes of chitosane nanofibers and cultured for increasing times. Observation of low magnification SEM pictures indicate that both cell types begin to attach and spread within one day, and then proliferate over time.

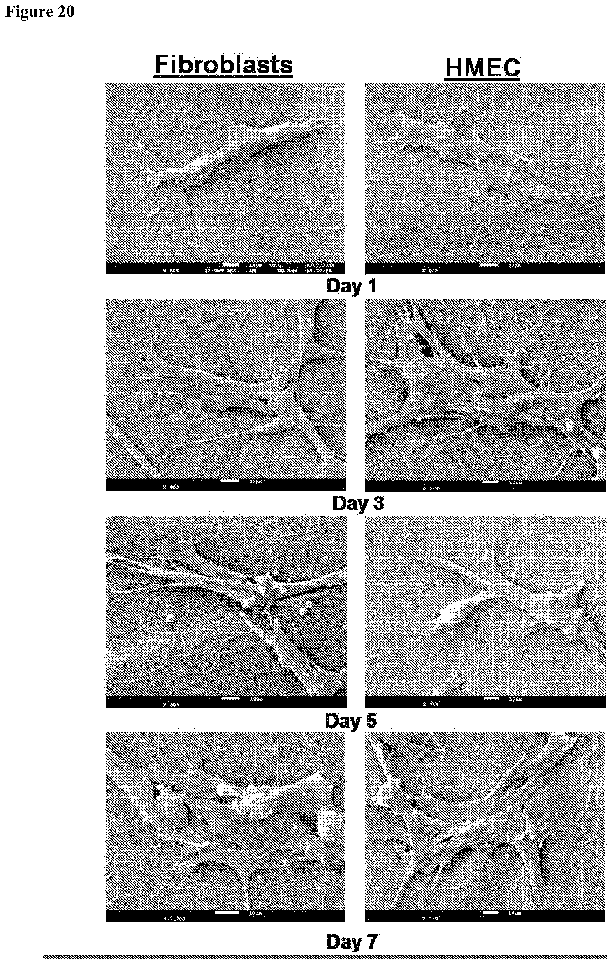

**Figure 20****. Observation by scanning electron microscopy (SEM) of cell attachment, spreading and proliferation.** Fibroblasts and HMEC were seeded on membranes of chitosan nanofibers and cultured for increasing times. Observations of intermediate magnification SEM pictures indicate that both cell types begin to attach and spread within one day. Better spreading and increased interactions with the nanofibers are observed at days 3 to 7.

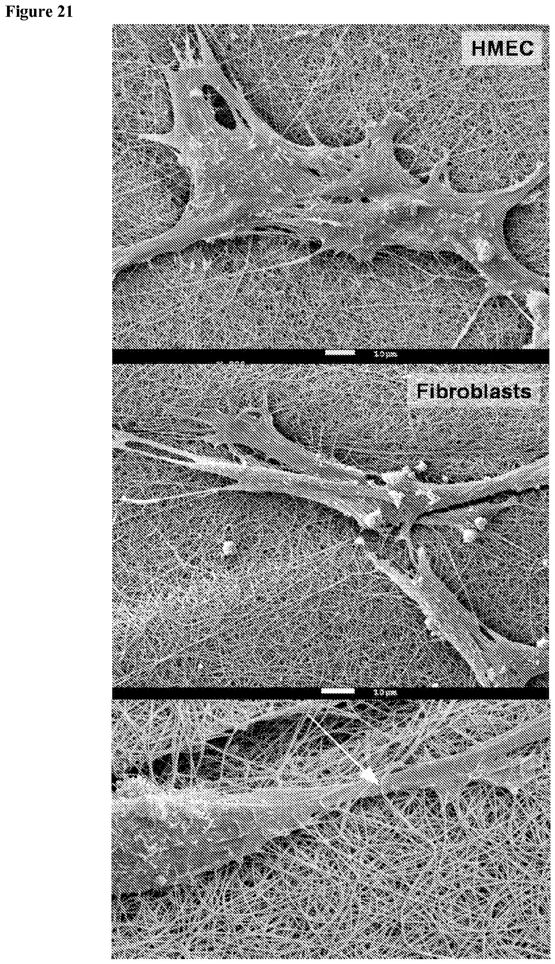

**Figure 21****. Observation by scanning electron microscopy (SEM) of cell attachment, spreading and proliferation.** Fibroblasts and HMEC were seeded on membranes of chitosan nanofibers. After few days of culture, observations of high magnification SEM pictures revealed close contact and interactions between chitosan nanofibers and cell filopodia and lamellipodia. In some specific area, cells seemed to start invading the three-dimensional scaffold of nanofibers (white arrow, third panel).

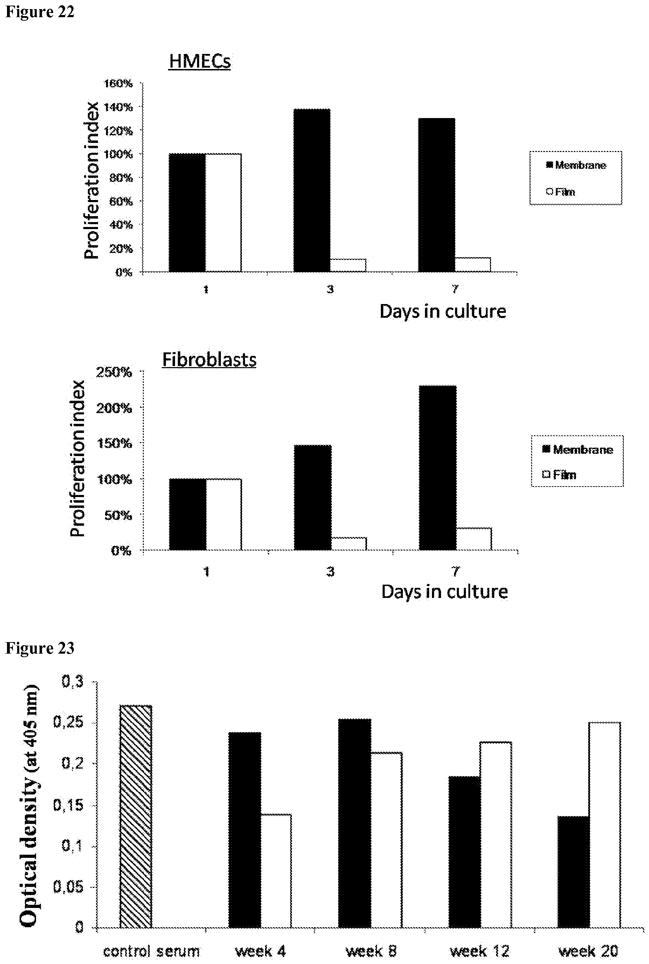

**Figure 22****. Evaluation of the proliferative index.** Fibroblasts and HMEC were cultured for increasing times on membrane of chitosane nanofibers and on chitosan film. Evaluation of the proliferation index was performed by measurement of the incorporation of [3H] thymidine into TCA-precipitable DNA. Day one was considered as being the 100 % reference. While both cell types proliferated on nanofiber membranes, a clear reduction of incorporated radioactivity was observed for cell on chitosane films, suggesting cell detachment or dying over the time in culture.
**Figure 23****: Evaluation by ELISA of the potential presence of anti-chitosan antibodies in the serum of mice implanted with chitosane biomaterial.** Control serum consisted in the serum of mice that have never been in contact with chitosane. Measurements for mice implanted with chitosan sponges or electrospun chitosan nanofibers are represented by black bar and open bars, respectively. Values observed for implanted mice are always in the range of the negative control and not affected by the structure of the biomaterial (lamellar sponges or nanofibers) or the duration of the implantation (4 to 20 weeks).

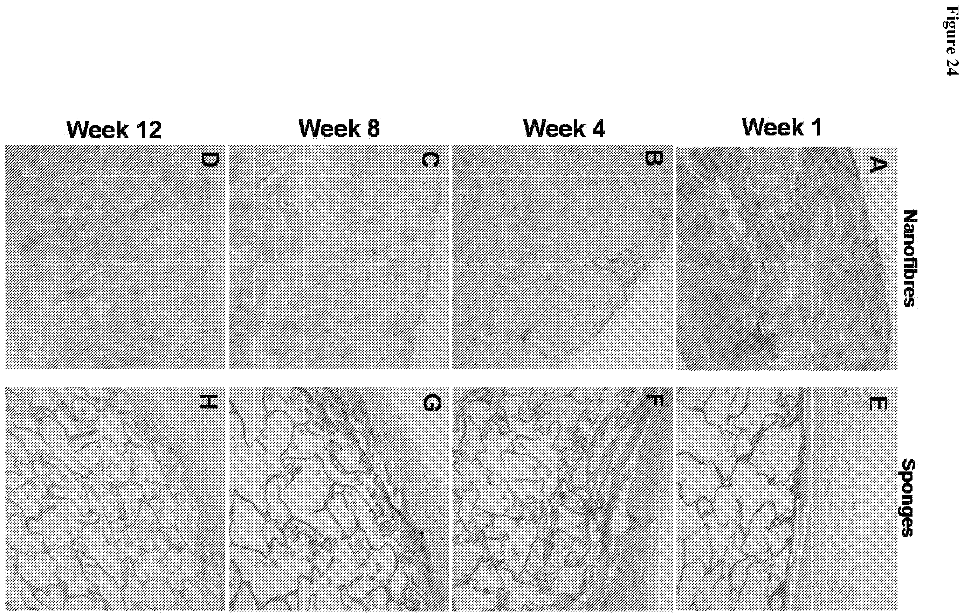

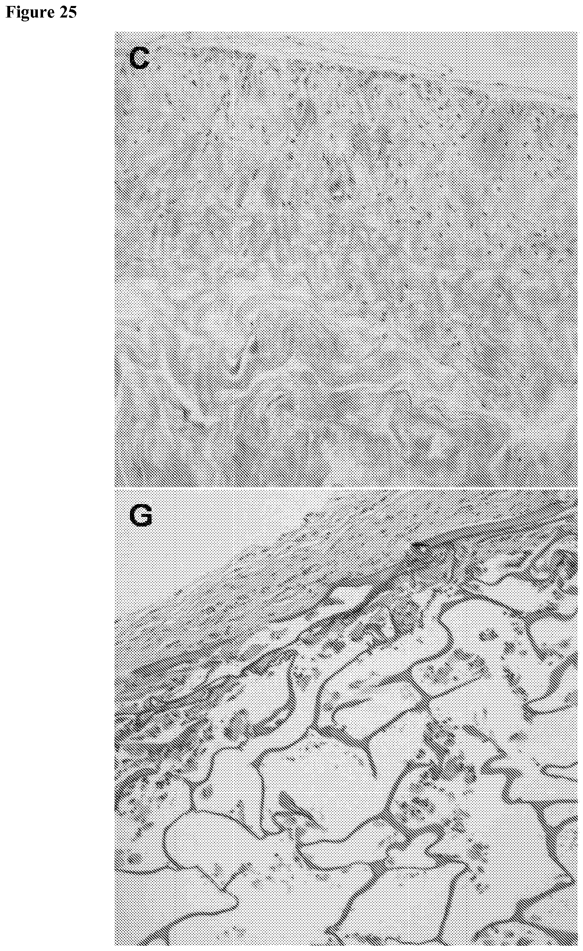

**Figure 24****: Hematoxylin-Eosin staining of electrospun chitosan membranes and sponges at increasing time after implantation in mice.** Electrospun chitosan membranes and sponges were implanted in the back of Balb/c mice and recovered after 1 to 12 weeks. After fixation and embedding in paraffin, sections were made, stained (hematoxylin/eosin) and visualized. Chitosan electrospun membranes appears as pink a undulating structure (A-D). In sponges (E-H) chitosan is in the form of a multi-lamellae structure. Cells are evidenced by the blue staining of the nuclei. They are progressively invading the electrospun membrane (A-D) while they stay in the periphery of sponges, forming a multilayer granuloma. This is more easily observed at a higher magnification (Figure 25).

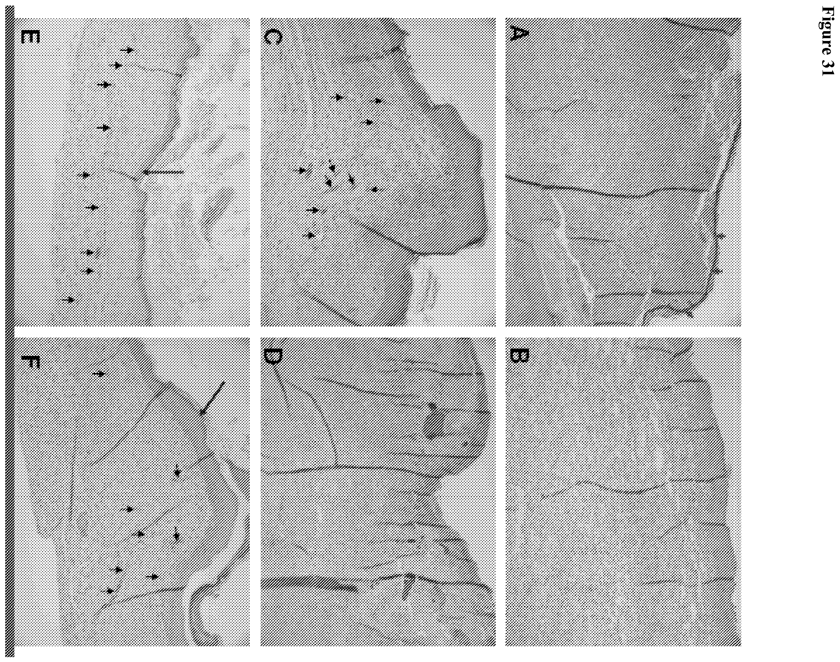

**Figure 25****: Hematoxylin-Eosin staining of electrospun chitosan membranes and sponges 8 weeks after subcutaneous implantation in mice.** These pictures are higher magnification of panels C and G of Figure 31. Cells invading the electrospun membranes are identified by the blue staining of the nuclei (C). No granuloma can be observed around the implanted biomaterial. For sponges (G) a peripheric granuloma is observed, while mesenchymal cells do not migrate inside the chitosan structure (the blue staining in the sponges being caused by fibrin deposit and the presence of inflammatory cells).

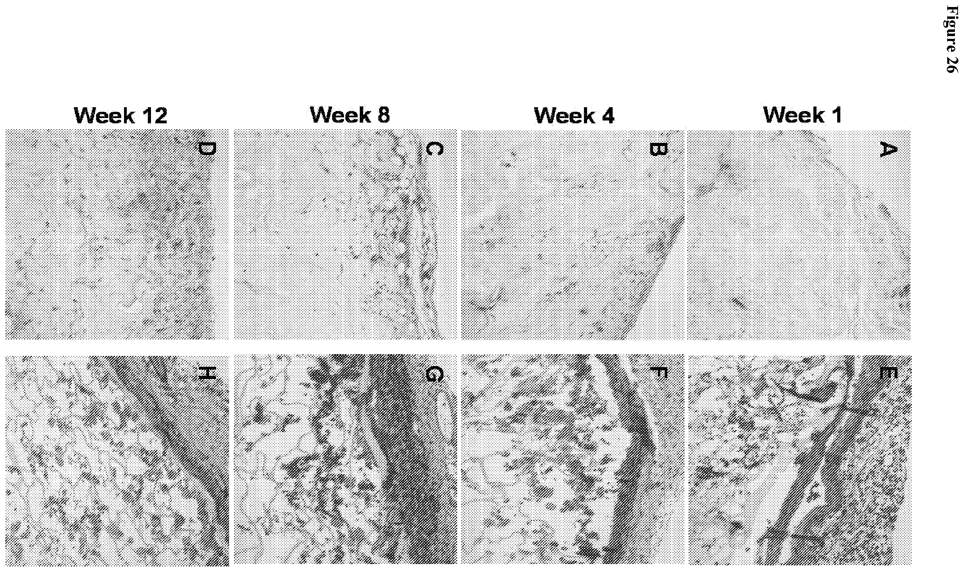

**Figure 26****: Leukocytes present in electrospun chitosan membranes and sponges 1 to 12 weeks after subcutaneous implantation** in **mice.** Immunostaing (in brown) of leukocytes was performed by using an anti-CD45 antibody. An excessive immune reaction is observed around and inside the sponges (E to H). At the contrary, a limit staining is observed in the electrospun membranes. Such a moderate immune reaction is usually considered as beneficial for an efficient healing or repair process.

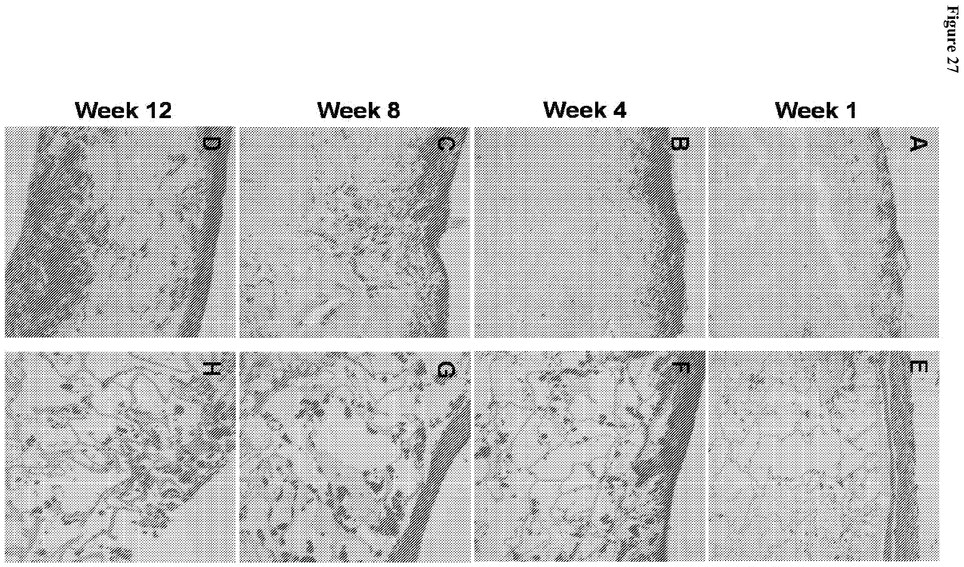

**Figure 27****: Staining of mesenchymal cells** in **electrospun chitosan membranes and sponges 1 to 12 weeks after subcutaneous implantation in mice.** Immunostaing of mesenchymal cells (in brown, essentially fibroblasts, myofibroblasts and smooth muscle cells) was performed by using an anti-vimentin antibody. A progressive invasion of the electrospun membrane by mesenchymal cells was clearly evidenced (A to D). By contrast, only the outer granuloma containing activated myofibroblats is labelled in the case of sponges (E to H).

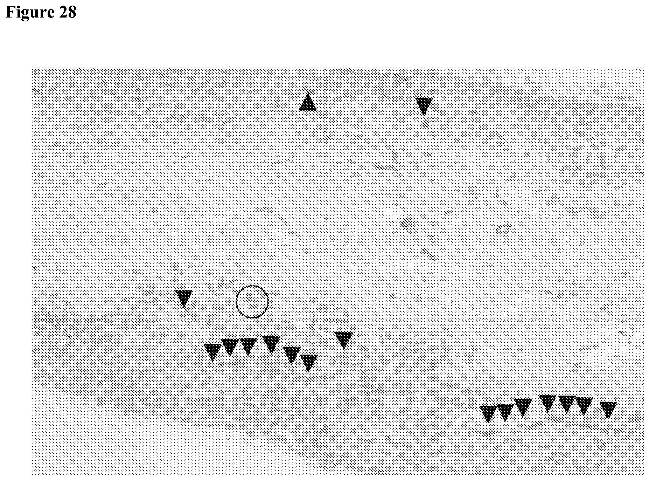

**Figure 28****: Staining of blood vessels in electrospun chitosan membranes 12 weeks after subcutaneous implantation in mice.** Immunostaing of the basement membrane present in established blood vessels was performed by using an anti-type IV collagen. Positive cells and structures are enlightened by arrowheads, showing blood vessel in longitudinal or cross section. A capillary with a lumen is circled.

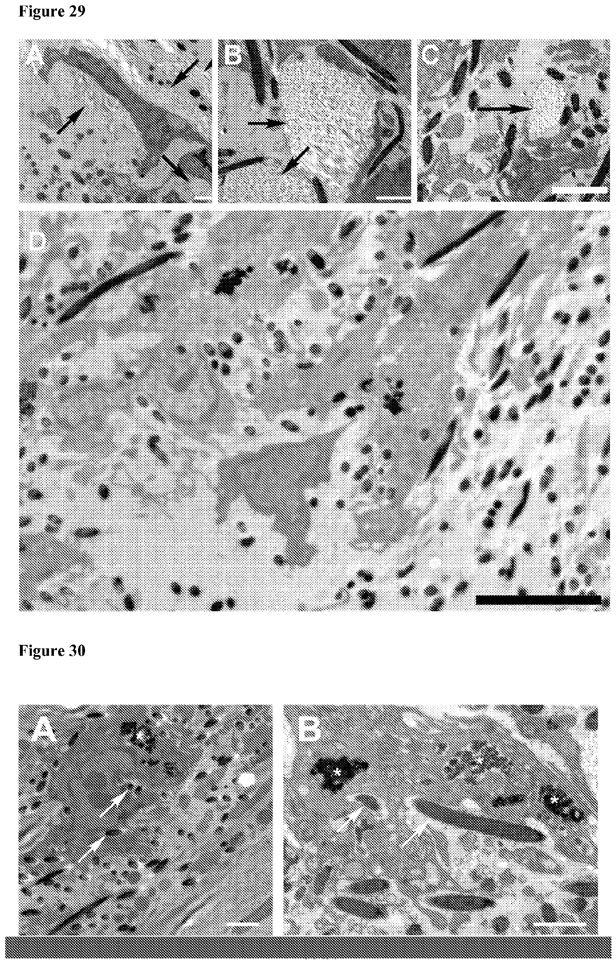

**Figure 29****: Transmission electron microscopy visualization of implanted electrospun chitosan membrane.** Electrospun chitosan membranes were subcutaneously implanted. After 12 weeks, the animals were sacrificed and the tissue containing the electrospun chitosan was fixed and processed for transmission electron microscopy. Chitosan nanofibers appear as black elongated cylindrical structure in longitudinal section and as black circles in cross sections. Numerous fibroblasts were identified (A) producing collagen fibers and fibrils appearing as bright structure (black arrows). These collagen fibers are in close association both with cells and chitosane nanofibers (A, B, C). A larger picture (C) shows the close and thigh association between the host cells and the scaffold of chitosan nanofibers. Bars: 5 µm (D) and 2 µm (A, B, C).
**Figure 30****: Chitosan fibers progressive degradation.** Implanted electrospun chitosan membranes were recovered 12 weeks after surgery, fixed and processed for transmission electron microscopy. Chitosan fibers are sometimes observed in contact with cell exhibiting characteristics of macrophages. Nanofibers of chitosan that seem to be phagocytised are indicated by white arrows. Black structures indicated by asterisks are probably chitosan being degraded. Bars: 2 µm (A) and 1 µm (B).
**Figure 31****: Hematoxylin and eosin-stained sections of biopsies for the morphological evaluation of skin lesions.** Wounds covered by electrospun chitosan nanofibres membranes at day 7 (A), day 14 (C), and day 21 (E). Control wound at day 7 (B), day 14 (D), and day 21 (F). Black arrow: Blood vessels; red arrow: electrospun chitosan nanofibres stuck to the wound; blue arrow: Epithelial layer.

The following non-limiting examples illustrate the invention:

### Example 1: Electrospinning of chitosan solution

### 1.1 Material:

Medical grade chitosan with acetylation degree 19.6%, viscosity (1% solution in 1% acetic acid) 21 mPa.s, Mw 77 kDa (L08049), from Kitozyme, Belgium and PEO high molecular weight (HMW, 900000 g/mol) purchased from Aldrich were used as received. Acetic acid, ethanol and sodium hydroxide (Sigma) were analytical grade and used as received.

### 1.2Method:

### 1.2.1 Electrospinning of chitosan membranes

Solutions of 10.5% chitosan, 4% HMW PEO were prepared by dissolving the appropriate amount of chitosan (in 6.5% acetic acid solution) and PEO (in distilled water), by stirring overnight. The next day, the chitosan and PEO solutions were then mixed to obtain mixtures with weight ratios of chitosan:PEO of 90:10. Two ml of the well homogenized resulting mixture were fed into 5ml plastic syringes fitted with blunt tipped stainless steel needles (gauge 18 and 21). The solution feed was driven using a syringe pump (Razel Scientific Instruments) and an electrospinning voltage ranging from 15 to 30 kV was applied between the needle and the collector (aluminium foil) by the use of a Spellman SL10 power supply. The positive electrode of a high voltage power supply was connected to a metal capillary by copper wires. The distance needle tip - collector was 15 cm, and the flow rate of the solution was 0.75 ml/h. All electrospinning experiments were performed at room temperature. The nanofibrous nonwoven mats were collected on the surface of aluminum foil.

### 1.2.2 Stabilization treatments

For improving the water stability of the electrospun material, the as-spun membranes were treated first with ethanol and then with NaOH (1M) solutions. Subsequently, the membranes were extensively rinsed with distilled water and dried under the vacuum.

### 1.2.3 Characterization techniques

To analyze the composition of electrospun and stabilized nanofiber mat, infrared spectra were recorded with a Perkin-Elmer FT-IR 1720X and differential scanning calorimetry (DSC) was carried out with a TA DSC Q100 thermal analyzer calibrated with indium. The electrospun nanofibers were sputter-coated with Pt and their morphology examined with a scanning electron microscope (Jeol JSM 840A).

### 1.3 Chitosan membranes:

The as-spun chitosan membranes prepared with the electrospinning method mentioned above are highly positively charged, thus they dissolve in acids and exhibit a low stability in neutral or weak alkaline aqueous media. As far as these membranes are foreseen in tissue engineering, their stability in cell culture and physiological media is required, This has been achieved in the present approach, by reducing the content of PEO necessary for electrospinning below 15%, by neutralizing the chitosan nanofibers after electrospinning and by removing of the PEO by repetitive washings steps. This stabilization process, i.e. without the use of chemical cross-linkers or chlorine-containing organic solvents. results in the long-term stability of the chitosan nanofibers in water and phosphate buffers. Stabilized membranes can be stored in distilled water for months without any apparent change of the morphology. (Figure 2)

### Example 2: Engineering of the dressing

As reported above, bandages and dressings for treating wounds have to satisfy various requirements. This explains why the conventional fibrous matrix scaffold obtained so far by electrospinning of chitosan is not used today. Some of these disadvantages are that:
a) a fibrous scaffold composed of chitosan nanofibers has poor mechanical properties that prevent easy handling and cause rapid destruction in situ when placed on wounds.
b) nanofibrous scaffolds having only 2-dimensional structure are limited in applications since they do not provide protection against outside mechanical influences.
c) thin nanofibrous scaffolds are not appropriate to maintain the moisture equilibrium promoting the wound healing process.
d) The pores in scaffold made of cross-linked nanofibers are too small to permit cell invasion, which, in certain circumstances, is a disadvantage.

The present invention overcomes all of these problems by providing a wound dressing consisting in multiple sheets or layers. The designation of a "first layer", "second layer", and the like, is meant to describe the location of a material relative to the wound bed. For example, the material located adjacent to the wound bed and in contact with it is termed the "first layer". The material that is placed on top of the first layer (proceeding in a direction away from the wound bed) is termed the "second layer", and so on. A layer may comprise one material, or two or more materials. In our prototype of dressing, the first layer is made of a non woven chitosan nanofibers mats obtained by electropinning (first layer) on an absorbent neutralized chitosan sponge (Kitozyme) or sponge from Hemcon (ChitoFlex™) being the second layer (Figure 3). The chitosan sponge used in this invention is porous, and has interconnecting pores having a pore size in the range of about 50-400 microns. When the nanofibers come to the surface of the sponge, they still contain acetic acid that dissolves the surface of the sponge. Although this process is very limited, it allows adhesion of nanofibers mat on the sponge without using any synthetic adhesive. Interactions between these two layers are strong enough for easy handling of the entire dressing but still loose enough for easy separation of the two layers if required for clinical applications (Figure 4).

Our dressing retains the flexibility required for optimal adhesion to the wound, although possessing adequate mechanical properties for easy handling. This dressing has also the capacity to absorb large amount of aqueous liquid, for instance exudates in the specific case of wounds.

The electrospinning process is capable of generating fibrous scaffolds from both natural and synthetic polymers. The limitation of scaffolds fabricated using the electrospinning process is the high fiber density and the resultant "fish net effect" (Figure 2). In other words, fiber density in electrospun mats is often too high to allow cell infiltration. The mean pore radius of electrospun matrices varies with fiber diameter. For example, a 100-nm fiber diameter yields a mean pore radius less than 10 nm at a relative density of 80%. The comparative size of a rounded cell - ranging from 5 to 20 µm - predicts that such small pore sizes would prevent cell infiltration or invasion. A number of methodologies for regulating pore size have been proposed. Recently, cells have been electrosprayed into forming scaffolds. However, issues of layering, sterility, and time to produce thick scaffolds are expected to limit application. A more efficient method for improving cell invasion may be by increasing porosity. Experiments with porous foams and sponges suggested that it exists an optimal pore size for cell infiltration. For fibrous scaffolds, this approach has been addressed by mixing fibers of different diameter. Alternatively, pores have been produced by including sacrificial fibers in a composite scaffold containing heterogeneous fibers that display different solubility in a given solvent: the selective removal of sacrificial fibers increasing porosity and accelerating cell infiltration.

The present invention demonstrates that freeze-drying is a rapid and easy way to provide electrospun membranes containing pores of desired size. While this process has been used to produce porous materials (foam, porous ceramics and the like), it has never been used for the creation of pores in as-spun membranes. After stabilisation treatment, dressing (nanofibers and foam) was washed and frozen at -20°C during 12h. During the freezing process growing ice crystals push back and concentrate the fibers into the inter-crystals space. After complete solidification, the frozen dressing is lyophilised (to sublimation of the ice under vacuum), resulting in a fibers mat with pores of the size and shape of the initial ice crystals (Figure 5). In this condition (-20°C during 12h) the pore size obtained in a fibers mat is, ∼50% of 1 to 2µm pore diameters, ∼30% of 5 to 7µm pore diameters and ∼10% of 10 to 15 pore diameters. (Figure 5) However, depending on the freezing conditions the size of the ice crystals can be modulated, regulating accordingly the size of the resulting pores in the nanofiber layer of the mat. Previously, there have been many studies that have investigated the structure of ice crystals formed in various kinds of materials and foods. Thus, it is well-known that rapid freezing rather than slow freezing gives smaller size ice crystals in frozen samples. Also, it has been reported that ice crystals grow in size during storage by recrystallization, depending on the storage time and temperature. This treatment will improve the rapid colonization of the dressing (when desired) and increase its permeability to air, oxygen, water vapour and liquid water.

This structure of dressing (nanofibers with pores onto a sponge) affords rapid absorption of exudates and thereby draws bacteria away from the wound, helping to protect against wound sepsis, as will be discussed below. Dressings of the present invention can provide water absorptive capabilities as high as about 24 grams of water per gram of dressing. It is by the nature of fluidic flow that exudates tend to fill the available volume before progression onto higher levels of the layers. As exudates move into the more voluminous pores of the second layer, the gating or inhibition to flow is reduced and exudates may flow into this new region more rapidly. The effect of this is to provide a cone-like spread to the exudates passage, that is to say exudates are spread over a greater area of the wound dressing upon its passage through the graded density layer 1. The exudate that passes through the first layer is rapidly absorbed by the absorbent layer 2. However, the wound site is still kept moist by the effect of the graduated density of layers. As the exudates are spread by this layer 1, even when the absorbent layer directly above the wound site becomes saturated, the exudates may still pass through and be absorbed at more inclined areas of the layer 2.

### Example 3: Co-electrospinning of chitosan with proteins and chemical molecules having the ability to maintain and to preserve the biological activity of proteins.

### 3.1. Materials:

The β-lactamase BlaP of *Bacillus licheniformis* were used as protein models in co-electrospinning experiments. This protein corresponds to a bacterial enzyme with a globular 3D-structure. The recombinant protein was overproduced overproduced in *E. coli* and purified to homogeneity by using affinity chromatography methods.

### 3. 2. Methods:

### 3.2.1 Co-electrospun chitosan-proteins based membranes:

For co-electrospinning experiments, the BlaP protein was added in the chitosan/PEO mixture (see Example 1) at a final concentration of 3 mg/ml and electrospun using the same procedure described in Example 1.2.1. In some cases, the mixture was also supplemented with a derivative of β-cyclodextrin.

### 3.2.2 Detection of proteins in co-electrospun chitosan membranes

For the β-lactamase assays performed with BlaP, the co-electrospun polymers were incubated during 4 minutes with 1 ml of 100 µM nitrocefin (a chromogenic β-lactam substrate). Then the absorbance of the hydrolyzed product was measured at 482 nm. This absorbance value is thus directly correlated to the active β-lactamase immobilized on the polymer.
**3.3 Results and discussion:** Chitosan:PEO mixtures were co-electrospun with the class A β-lactamase BlaP, with a derivative of β-cyclodextrin or with these two products together, for investigating the ability of the protein to maintain its post-electrospinning activity, as well as the capability of cyclodextrin to stabilise the protein in the due conditions. The conditions for electrospinning were: 30 kV voltage, 15 cm distance between electrodes, 0.762 ml/h solution flow rate, 30 min electrospinning time. Adding cyclodextrin alone to the chitosan:PEO mixture increased the incidence of spindle-shaped fibers, while adding protein contributed to the production of smooth, bead-free fibers, irrespective of the presence or absence of cyclodextrin in the electrospinning mixture. In addition, we also attempted to measure the β-lactamase activity immobilized on the co-electrospun chitosan polymers obtained in the absence and in the presence of cyclodextrin. The data are presented in Figure 6. The rate of nitrocefin hydrolysis (ΔA/4min/mg of polymer) obtained in the presence and in the absence of cyclodextrin were 0.939 ± 0.125 and 0.577 ± 0.111, respectively. These ones indicate that the addition of β-cyclodextrin increases significantly (+39%) the β-lactamase activity recovered in the co-electrospun product. In both cases, we also monitored the immobilized activity during storage of the polymers at room temperature after 6 weeks. No decrease of the immobilized β-lactamase activity was observed neither in absence nor in presence of cyclodextrin.

### Example 4: Conversion of chitosan nanofibers into chitin with preservation of the nanofibers morphology

### 4.1 Materials

A medical grade chitosan of fungi origin (kiOmedine-CsUP™, Kitozyme, Belgium) was used. Its degree of acetylation (DA) and molecular mass (MM) were 19% and 68000, respectively. All chemical and biochemical reagents were of analytical grade.

### 4.2 Covalent immobilisation of chitosan on Immobilizer ^{Tm}-Amino modules/plates

Thirty mg of chitosan were solubilized in 2.5 ml of 0.1 M Sodium acetate (pH3). After an overnight incubation under stirring condition at room temperature, the chitosan solution was diluted 10x with 0.1M phosphate (pH7.4). Next, 150µl of this chitosan solution were added in each well of an Immobilizer Tm-Amino modules/plates (Exiqon A/S) for 2h at room temperature. Covalent bonds between the activated groups of the well and the amine groups of chitosan are established. The plates are next washed 3 times with 20mM Tris (pH8) by using a plate washer Biotrak II (Amersham Biosciences). To block the residual activated groups that have not reacted with chitosan or Tris, two washes of 20 min each are performed with 250µl/well of 20mM Tris (pH8).

For chitosan binding assays, the wells were filled with 200µl of PBS containing 3%BSA and incubated overnight at 4°C. The plates were next washed 3 times with PBS (50mM phosphate, 150mM NaCl, pH7.4) by using a plate washer Biotrak II.

### 4.3 Chemical conversion of immobilized chitosan on Immobilizer ^{Tm}-Amino modules/plates

Thirty mg of chitosan were solubilized in 2.5 ml of 0.1 M Sodium acetate (pH3). After an overnight incubation under stirring condition at room temperature, the chitosan solution was diluted 10x with 0.1M phosphate (pH7.4). Next, 150µl of the new chitosan solution were added in each well of Immobilizer Tm-Amino modules/plates (Exiqon A/S) for 2h at room temperature. Covalent bonds between the activated groups of the well and the amine groups of chitosan are established. Then, 99% pure acetate anhydride (10.4 M) was diluted in different organic solvents and 60µl/well of these preparations were added to the wells filled with the chitosan solution. All the acetate anhydride preparations were tested individually for the reacetylation of chitosan. The reacetylation of chitosan was conducted for 1h at room temperature under stirring condition. The plates were next washed 3 times with PBS (50mM phosphate, 150mM NaCl, pH7.4) by using a plate washer Biotrak II.

For chitin binding assays, the wells were filled with 200µl of PBS additionned with 3%BSA and incubated overnight at 4°C. The plates were next washed 3 times with PBS (50mM phosphate, 150mM NaCl, pH7.4) by using a plate washer Biotrak II.

### 4.4 BlaP ChBDAl construction and expression

The gene coding for the chitin-binding domain of *Bacillus circulans* WL-12 chitinase A1 (ChBDA1) was PCR amplified using Pfu polymerase with primers CHBDA1+ and CHBDA1- (5'-GGAACGACAAATCCTGGTGTATCCGCTTGGCAGGTC-3' (SEQ ID NO:1) and (5'-TCCTTGAAGCTGCCACAATGCTGGAACGTTGGATGG-3' (SEQ ID NO:2)). The PCR products were successively purified on a GFX™ gel band purification kit (Amersham Biosciences, UK), phosphorylated using T4 polynucleotide kinase and purified again with GFX™ gel band purification kit before to be cloned into the *SmaI*-digested and dephosphorylated pNY-ESBlap. By using this vector, a full constitutive expression of the β-lactamase BlaP is obtained in *E. coli.* The resulting genetic construct was called pNYBlapChBDA1. In this construction, the β-lactamase BlaP sequence is preceded by a signal peptide for periplasmic secretion and followed by a (his)₆-tag on the C-terminal end to improve its purification by affinity chromatography. The nucleotidic sequence encoding ChBDA1 is cloned into the nucleotidic sequence of BlaP. The chitin binding domain is expressed in a loop of the β-lactamase BlaP which is solvent accessible and diametrically opposed to the active site of the enzyme to avoid steric hindrance (Figure 7). This technology refers to patent EP1713907 (Hybrid protein of active-site serine β-lactamase). To achieve production of the hybrid β-lactamase BlapChBDA1, *E. coli* JM109 transformed respectively with pNYBlapChBDA1, were grown in Terrific Broth supplemented with 75 µg/ml spectinomycin and 10 µg/ml ampicillin at 37°C. Cells from an overnight culture (1L) were harvested by centrifugation (9000 g for 15 min) and resuspended in 40 ml of TES (20% sucrose, 30 mM Tris-HCl, 5 mM EDTA, pH 8) at 37°C. The bacterial suspension was placed under stirring at 37°C for 10 min. Cells were harvested by centrifugation (9000 g for 15 min) and the pellet resuspended in 100 ml of 5 mM MgSO₄ at 4°C. The bacterial suspension was stirred at 4°C for 10 min. The supernatant containing the periplasmic proteins was harvested by centrifugation (13000 g for 20 min) and diluted with three volumes of 50 mM phosphate (pH 7.4). The periplasmic proteins were loaded on a HisTrap™ Chelating HP column (GE Healthcare) equilibrated in 50 mM phosphate (pH 7.4). The column was successively washed with 2M NaCl and 50 mM phosphate (pH 7.4) supplemented with 10 mM imidazole. The hybrid proteins were eluted by an imidazole linear gradient (10 to 500 mM) in 50 mM phosphate (pH 7.4). Fractions containing the purified hybrid proteins were pooled and dialyzed against PBS (50 mM phosphate, 150 mM NaCl, pH 7.4).

### 4.5 BlaP ChBDhmc construction and expression

The gene coding for the chitin-binding domain of the human macrophage chitotriosidase (ChBDhmc) was constructed by overlapping PCR and cloned into the pGEM-T-easy vector (Promega) for sequencing. The oligonucleotides used in the overlapping PCR are presented in Table 1 (ChitO1 to ChitO8).

**Table 1. Oligonucleotides used in the construction of BlaP ChBDhmc.**

| | |
|---|---|
| ChitO1 | GCCATGGGACCAGAGCTTGAAGTTCCTAAACCAGGACAGCCCTCTGAACCTGAGCATGGC(SEQ ID NO:3) |
| ChitO2 | CCCTCTCCAGGACAAGACACGTTCTGCCAGGGCAAAGCTGATGGGCTCTATCCTAATCCT(SEQ ID NO:4) |
| ChitO3 | CGTGAACGGTCCAGTTTCTATAGTTGTGCTGCAGGTCGGCTGTTCCAACAAAGTTGTCCAACAGGTCTGG(SEQ ID NO:5) |
| ChitO4 | GGGATCCATTCCAGGTACAACATTTGCAGGAGTTGCTGAACACCAGACCTGTTGGACAACTTTGTTGGAACAGCCG(SEQ ID NO:6) |
| ChitO5 | ACCTGCAGCACAACT A T AGAAACTGGACCGTTCACGAGGA TT AGGA T AGAGCCCATCAGCTTTGCCCTGGC(SEQ ID NO:7) |
| ChitO6 | AGAACGTGTCTTGTCCTGGAGAGGGGCCATGCTCAGGTTCAGAGGGCTGTCCTGG(SEQ ID NO:8) |
| ChitO7 | GCCATGGGACCAGAGCTTGAAGTTCCTAAA(SEQ ID NO:9) |
| ChitO8 | GGGATCCATTCCAGGTACAACATTTGCAGGAG(SEQ ID NO:10) |
| CHIT1+ | GGACCAGAGCTTGAAGTTCCTAAACCAGGACAGCCCTCT(SEQ ID NO:11) |
| CHIT1- | TCCATTCCAGGTACAACATTTGCAGGAGTTGCTGAACACCAGACC(SEQ ID NO:12) |

Next, the gene coding for ChBDhmc was PCR amplified using Pfu polymerase with primers CHIT1+ and CHIT1-. The PCR products were successively purified on a GFX™ gel band purification kit (Amersham Biosciences, UK), phosphorylated using T4 polynucleotide kinase and purified again with GFX™ gel band purification kit before to be cloned into the Smal-digested and dephosphorylated pNY-ESBlap. The resulting genetic construct was called pNYBlapChBDhmc.

To achieve production of the hybrid β-lactamase BlapChBDhmc, *E. coli* JM109 transformed respectively with pNYBlapChBDhmc were grown in Terrific Broth supplemented with 75 µg/ml spectinomycin and 10 µg/ml ampicillin at 37°C. Cells from an overnight culture (1L) were harvested by centrifugation (9000 g for 15 min) and resuspended in 40 ml of TES (20% sucrose, 30 mM Tris-HCl, 5 mM EDTA, pH 8) at 37°C. The bacterial suspension was placed under stirring at 37°C for 10 min. Cells were harvested by centrifugation (9000 g for 15 min) and the pellet resuspended in 100 ml of 5 mM MgSO₄ at 4°C. The bacterial suspension was stirred at 4°C for 10 min. The supernatant containing the periplasmic proteins was harvested by centrifugation (13000 g for 20 min) and diluted with three volumes of 50 mM phosphate (pH 7.4). The periplasmic proteins were loaded on a HisTrap^{Tm} Chelating HP column (GE Healthcare) equilibrated in 50 mM phosphate (pH 7.4). The column was successively washed with 2M NaCl and 50 mM phosphate (pH 7.4) supplemented with 10 mM imidazole. The hybrid proteins were eluted by an imidazole linear gradient (10 to 500 mM) in 50 mM phosphate (pH 7.4). Fractions containing the purified hybrid proteins were pooled and dialyzed against PBS (50 mM phosphate, 150 mM NaCl, pH 7.4).

### 4.6 Chitosan and chitin binding assays on Immobilizer ^{Tm}-Amino modules/plates

To perform the chitosan and chitin binding assays, we used the plates described in points 2 and 3 of this Example. The binding assays were performed by using the hybrid β-lactamase BlaP ChBDA1 and BlaP ChBDhmc. To attest that the interaction of these hybrid proteins with chitosan is specific, we used the hybrid β-lactamase BlaP Actev as a negative control. The engineered loop of BlaP Actev does not contain chitin-binding domain but two Actev-cleavage sites surrounding the insertion site.

One hundred µl of purified β-lactamase diluted in PBS (5µg/ml) were added to the coated wells and the plates were incubated for 2h at room temperature. Next, the plates were washed 3 times with PBS. The direct measurement of the immobilized β-lactamase activity in each well was done by following the hydrolysis of 150 µl of nitrocefin (100µM) in 50 mM phosphate buffer (pH 7.5) at 482 nm.

### 4.7 Electrospun membranes

Membranes formed by electrospun chitosan nanofibers were prepared as described in Example 1. To improve the water stability of the electrospun material, the as-spun membranes were first soaked successively in pure ethanol and later with 1M NaOH. Next, the membranes were extensively rinsed with distilled water.

### 4.8 Chemical conversion of electrospun chitosan nanofibers into chitin

The electrospun nanofibrous membranes of chitosan were cut into square of 1cm² for chemical conversion assays. The samples were placed into closed tubes containing the differents acetate anhydride solutions presented in table 2. The polymers were incubated 1h at room temperature. Next, the polymer were extensively washed in water bath and dried into a dessicator before processing for scanning electron microscopy. In this work, we avoided air drying the polymers because this process caused a dramatic loss of the fibrillary structure of the polymer.

### 4.9 Scanning electron microscopy

The morphology of the electrospun and re-acetylated chitosan nanofibers were sputter-coated with Pd and examined with a scanning electron microscop (Jeol JSM 840A).

### 4.10 Expression and purification of the human lysozyme

Recombinant human lysozyme was generated using the pPIC9K *Pichia pastoris* expression vector (Invitrogen). Briefly, the gene encoding the lysozyme (P61626) was subcloned from the pGA HMlyso plasmid into pPIC9K vector (Invitrogen) by using the *SnaBI* and *NotI* restriction sites. The pPIC9K plasmid contains the α-factor secretion signal that directs the recombinant protein into the secretory pathway. The constructs were digested with *Sal* I and used to transform *Pichia pastoris* strain SMD168 by electroporation. This resulted in insertion of the construct at the AOX1 locus of *Pichia pastoris,* generating a His⁺ Mut⁺ phenotype. Transformants were selected for the His⁺ phenotype on 2% agar containing regeneration dextrose biotin (1 M sorbitol, 2% dextrose, 1.34% yeast nitrogen base, 4 x 10⁻⁵ percent biotin, and 0.005% of L-glutamic acid, L-methionine, L-lysine, L-leucine, and L-isoleucine) medium and then further selected for high copy number by their ability to grow on 2% agar containing 1% yeast extract, 2% peptone, 2% dextrose medium, and the antibiotic G418 at various concentrations (0.5-4 mg/ml) (Invitrogen). The protein was expressed in a shaker flask and harvested at 72 h after induction by methanol.

The protein was purified by using first a cation-exchange chromatography as follows: the supernatant was dialysed against buffer A (citrate 25mM, pH5) and applied onto a SP-Sepharose column equilibrated with buffer A. The column was washed with the same buffer. Elution was performed by an increasing linear (0-1M) NaCl gradient in buffer A with ten column volumes. The elution fractions were analysed on SDS-PAGE and the fractions containing the protein of interest were pooled and dialysed against buffer A. The protein was next applied onto a Puros 20HS column equilibrated with buffer A. The column was washed with buffer A. Elution was performed by an increasing linear (0- 1M) NaCl gradient in buffer A with ten column volumes. The elution fractions were analysed on SDS-PAGE. The fractions containing the protein of interest were pooled, dialysed against water and lyophilised.

### 4.11 Expression and purification of the human macrophage chitotriosidase

Recombinant human chitotriosidase was generated using the pPIC9K *Pichia pastoris* expression vector (Invitrogen). The gene encoding the chitotriosidase (GenBank, gi:4502808) followed by a His₆-tag on the C-terminus end was subcloned from the pGA HMchito plasmid into pPIC9K vector (Invitrogen) by using the *SnaBI* and *NotI* restriction sites. The pPIC9K plasmid contains the α-factor secretion signal that directs the recombinant protein into the secretory pathway. The constructs were digested with *Sal* I and used to transform *Pichia pastoris* strain SMD168 by electroporation. This resulted in insertion of the construct at the AOX1 locus of *Pichia pastoris,* generating a His⁺ Mut⁺ phenotype. Transformants were selected for the His⁺ phenotype on 2% agar containing regeneration dextrose biotin (1 M sorbitol, 2% dextrose, 1.34% yeast nitrogen base, 4 x 10⁻⁵ percent biotin, and 0.005% of L-glutamic acid, L-methionine, L-lysine, L-leucine, and L-isoleucine) medium and then further selected for high copy number by their ability to grow on 2% agar containing 1% yeast extract, 2% peptone, 2% dextrose medium, and the antibiotic G418 at various concentrations (0.5-4 mg/ml) (Invitrogen). The protein was expressed in a shaker flask and harvested at 72 h after induction by methanol.

The protein was purified by using nickel-nitrilotriacetic acid-agarose (Ni-NTA; Qiagen) as follow: the supernatant was applied onto a Ni-NTA-Sepharose column ((Novagen, USA, 1 x 10 cm) equilibrated with buffer A. The column was washed with seven column volumes of the same buffer, three column volumes of buffer A + 2 M NaCl and three column volumes of buffer A + 10 mM imidazole. Elution was performed by an increasing linear (10-500 mM) imidazole gradient in buffer A. Active fractions eluted at 100 mM imidazole and appeared as a single band upon SDS-PAGE analysis.

### 4.12 In vitro degradation of electrospun chitosan nanofibers

The nanofibrous membranes of chitosan and re-acetylated chitosan were cut into square of 1cm² for in vitro degradation testing. The samples were placed into closed tubes containing human lysozyme, human macrophage chitotriosidase or a mixture of human lysozyme / human macrophage chitotriosidase. The human lysozyme and the human macrophage chitotriosidase were used at a final concentration of 3.5 and 0.35 µM in phosphate buffer saline (pH 7.4), respectively.. The degradation assays were monitored during 6 weeks. Every three days, the enzymatic solution was replaced with a fresh solution. At specified time intervals, the electrospun membranes were taken out from the solution, washed with distilled water, dried, and weighted. Electrospun membranes were also incubated in phosphate buffer without enzyme to determine exactly the enzyme-catalysed degradation. The degree of *in vitro* degradation was expressed as the percentage of the dried sample weight before and after degradation.

### 4.13 Results

The goal of this study consisted to convert electrospun chitosan nanofibers to chitin nanofibers according to a process which permits:
- To maintain both the fibrillar structure of the nanofibers and the morphology of the biopolymer
- To restore the sensitivity to human glycosylhydrolases
- To control the reacetylation rate
- To combine chemical, biophysical and biological properties of chitosan and chitin in the same biopolymer

The chemical conversion of chitosan consisted to substitute the C2 amine (-NH2) group by an acetamide group (-NHCOCH3) with acetate anhydride (Figure 8).

First, we started to study the chemical conversion of chitosan with chitosan covalently immobilized on Immobilizer ^{Tm}-Amino modules/plates. Different acetate anhydride solutions were prepared and tested for the chemical conversion of chitosan (table 2).

**Table 2. Acetate anhydride solutions tested for the reacetylation of chitosan.**

| ***Ac anhydride* /*Organic solvent (Vol*/*Vol)*** | **Ethanol** | **Methanol** | **Acetone** | **Dimethyl Formamide** | **Heptane** | **Tetra-hydrofurane** |
|---|---|---|---|---|---|---|
| **Acetate anhydride** | 1/2 | 1/2 | 1/2 | 1/2 | 1/2 | 1/2 |
| | 1/4 | 1/4 | 1/4 | 1/4 | 1/4 | 1/4 |
| | 1/8 | 1/8 | 1/8 | 1/8 | 1/8 | 1/8 |
| | 1/16 | 1/16 | 1/16 | 1/16 | 1/16 | 1/16 |
| | 1/32 | 1/32 | 1/32 | 1/32 | 1/32 | 1/32 |
| | 1/64 | 1/64 | 1/64 | 1/64 | 1/64 | 1/64 |
| | 1/128 | 1/128 | 1/128 | 1/128 | 1/128 | 1/128 |
| | 1/256 | 1/256 | 1/256 | 1/256 | 1/256 | 1/256 |

To check the efficiency of the chemical conversion, chitin-binding assays were performed by using two hybrid β-lactamases harbouring the chitin-binding domains ChBDA1 or ChBDhmc. ChBDA1 and ChBDhmc were isolated from the *Bacillus circulans* WL-12 chitinase A1 and the human macrophage chitotriosidase, respectively. The hybrid β-lactamase BlaP Actev was used as a negative control to attest that the binding to chitin was only due to the presence of the chitin-binding domain into BlaP. The results are presented in table 3 and 4.

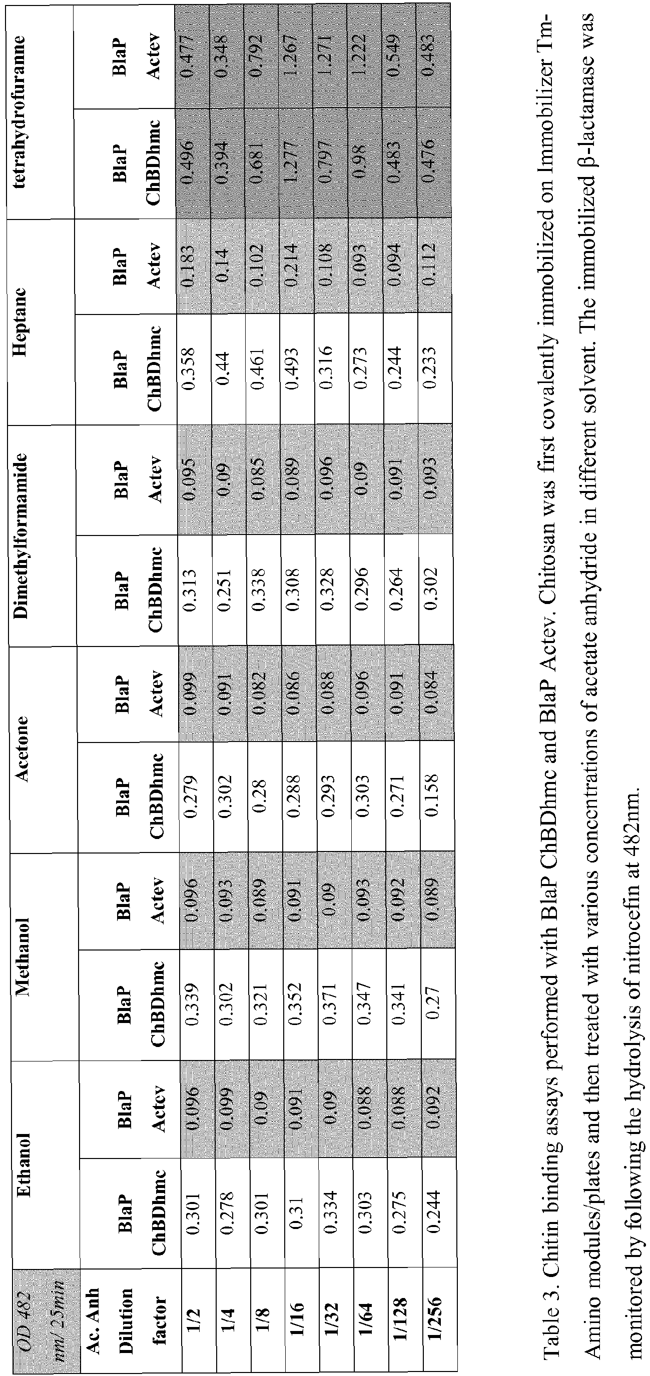

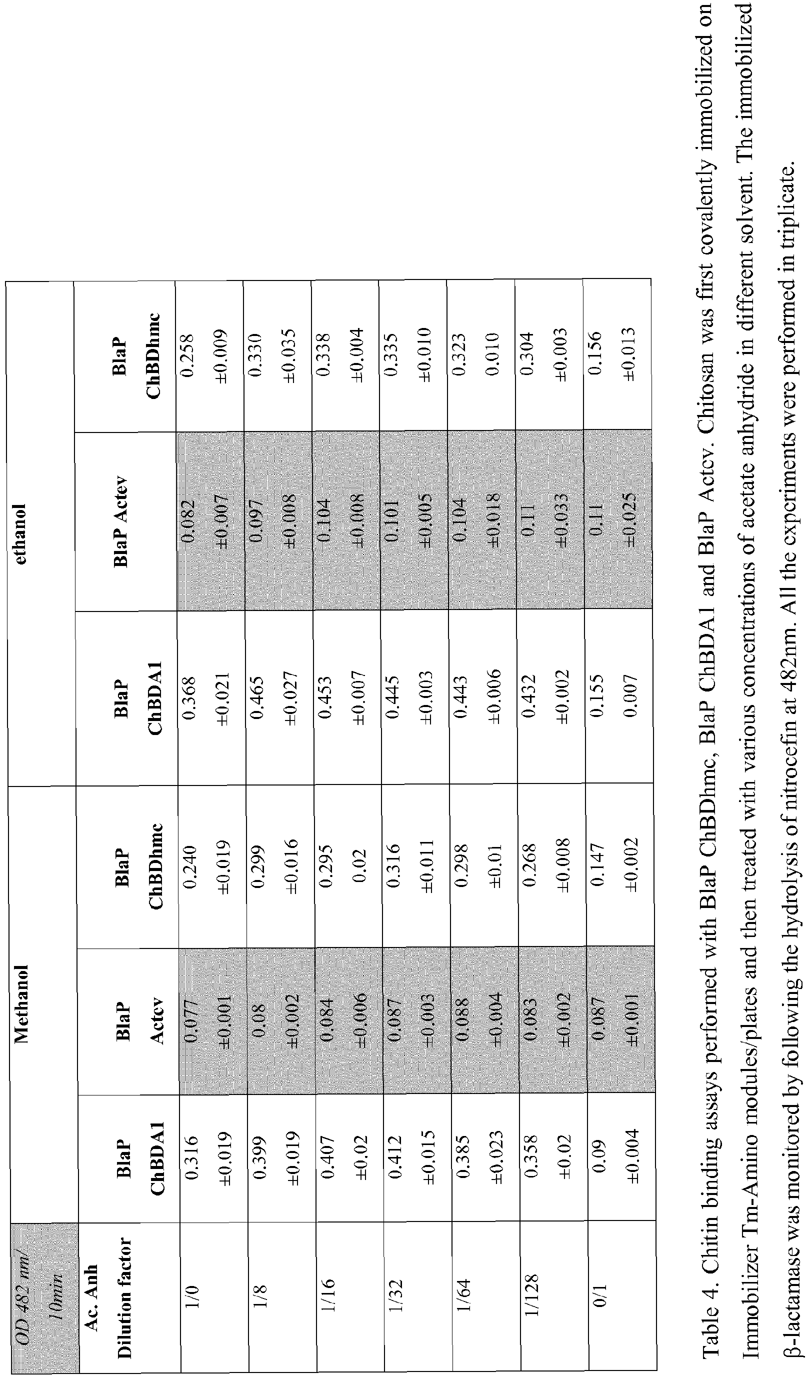

In regard to table 3, we demonstrated that the hybrid protein BlaP ChBDhmc detected the presence of chitin when the immobilized chitosan was treated with anhydride acetate diluted in ethanol, methanol, acetone, dimethylformamide or heptane whatever the dilution tested in a range from 1/2 to 1/ 256. No specific binding was detected with BlaP ChBDhmc when chitosan was treated with acetate anhydride diluted in tetrahydrofuranne. Globally, the most elevated chitin-binding activities were observed when acetate anhydride was diluted with ethanol and methanol in a large range of dilutions.

In the continuity of this work, we decided to focus on the chemical conversion of chitosan to chitin by using acetate anhydride diluted in ethanol and methanol. The results presented in table 4 were done in triplicate and confirmed the efficiency of acetate anhydride diluted in methanol and ethanol to convert chitosan to chitin.

The next step of this work consisted to check if the treatments described above permit also to convert electrospun chitosan nanofibers to chitin nanofibers without disturbing the fibrillary structure of the nanofibers and the morphology of the biopolymer. The electrospun and reacetylated chitosan nanofibers were sputter-coated with Pt and examined with a scanning electron microscop. The results are presented in Figure 9 and 10. In regard to these figures, we observed that the fibrillary structure of the electrospun re-acetylated nanofibers and the global morphology of the biopolymer were preserved only in some conditions. These conditions were limited to the use of methanol as organic solvent in a confined range of acetate anhydride dilutions from 1/16 to 1/64. All the conditions tested in the presence of ethanol resulted on the complete dislocation of the nanofibers, a drastic decrease of the porosity and the formation of a film.

We also checked if the hybrid β-lactamase BlaP ChBDA1 was also able to bind electrospun chitosan nanofibers when these ones are reacetylated with acetate anhydride diluted in methanol. The graph of Figure 11 attests that the chemical conversion in the presence of methanol results on the formation of chitin nanofibers because a specific binding of BlaP ChBDA1 is only observed when the chitosan nanofibers are treated. No binding was observed with BlaP Actev confirming that the binding of BlaP ChBDA1 was specific and related to the presence of chitin.

Finally, we compared the biodegradation of electrospun chitosan nanofibers before and after treatment with acetate anhydride diluted in methanol (1/32). The weight of the biopolymer was evaluated over a period of 6 weeks. The biopolymers were incubated in the presence of human lysozyme, human macrophage chitotriosidase and a mix of the two enzymes. The protein solutions were changed every two days. The histogram of Figure 12A shows the weak efficiency of human glycosylhydrolases to degrade alone or in combination the electrospun chitosan nanofibers. In Figure 12B, the histogram indicates that the treatment with acetate anhydride diluted in methanol (1/32) increases drastically the susceptibility of the nanofibers to the hydrolytic activities of human lysozyme and human macrophage chitotriosidase.

### Example 5: Characterization of normal human keratinocytes cultured on electrospun chitosan membranes

### 1. METHODS:

### Keratinocyte cultures:

Human keratinocytes were isolated by trypsin float technique [11] from normal adult skin samples. In order to study a representative cell population, the keratinocytes from three different donors were mixed in a keratinocyte pool. The pool was stored frozen in liquid nitrogen. The cell cultures were plated at density 30000 cells/cm² and incubated in KGM-2 keratinocyte medium at 37°C and 5% CO₂ in a humidified incubator. The medium was renewed every two days.

### Scanning electron microscopy:

Scanning electron microscopy (SEM) was used to analyze the adhesion, spreading and morphology, of keratinocytes cultured on electrospun chitosan membranes. The membranes were cut out with a punch (20mm in diameter) and put into 24-well culture plates, sterilized with 70% ethanol, air-dried in sterile culture hood and equilibrated with culture medium. Keratinocytes were seeded on electrospun chitosan membranes at a high cell density (30 000 cells/cm²) in KGM-2 medium. The cell cultures grown over electrospun chitosan membranes for various periods of time were fixed with 2.5 % glutaraldehyde for 20 minutes at room temperature, and then washed for 5 minutes with 0.1 M cacodylate buffer pH 7.4. The membranes were then dehydrated with rising concentrations of ethanol (25%, 50%, 75%, 95% and 100%), and then were subjected to critical point drying. The membranes were sticked to a support for scanning electron microscopy and then covered with a thin gold layer. The samples were examined and photographed in a Phillips XL20 scanning electron microscope.

### Histological analysis and hematoxilin staining:

For this analysis, keratinocytes cultured over electrospun chitosan membranes were fixed with 4% paraformaldehyde for 10 minutes, then subjected to dehydration in methanol and toluol, and then embedded vertically into paraffin. Then 6µm-thick paraffin sections were sliced and mounted onto glass slides. After removal of paraffin, sections were used for histological staining with hematoxilin.

### Phenotype analysis:

To analyse the phenotype of keratinocytes cultured on electrospun chitosan membranes, we have extracted total RNA after 7, 14 and 21 days in culture with the standard phenolchloroform procedure for RNA extraction, using the TRI Reagent® (Molecular Research Center, Inc., USA) and chloroform (Merck, Germany). After reverse transcription of RNA with Super Script II RNase H-Reverse transcriptase kit (Invitrogen, Belgium), we have performed real-time quantitative PCR for the analysis of the expression of specific genes (keratins 14 and 10, involucrin). The cDNAs were amplified using Power SYBR Green PCR Master Mix (Applied Biosystems, Belgium) and primer sense and antisense sequences (Sigma-Aldrich, Belgium) in a 7300 real- time PCR machine (Applied Biosystems, Belgium). mRNA levels were normalized to 36B4 (house-keeping gene) mRNA levels determined using the same procedure.

### 2. RESULTS AND DISCUSSION:

The first in vitro experiments were intended to analyse adhesion and proliferation of keratinocytes cultured on electrospun chitosan membranes. Because of the presence of the nanofibers forming the membranes, it was difficult to observe the growth of keratinocytes using phase-contrast microscopy, explaining why most of the observations were performed by scanning electron microscopy (SEM). The SEM images illustrate that normal human keratinocytes were capable to attach to chitosan nanofiber membranes, to proliferate, and reach cell confluence on the top of the membrane (Figure 13). No penetration of the cells into the nanofiber membrane was observed, probably because of the small spaces left between the fibers forming the membranes. An interesting observation was that freshly seeded keratinocytes formed lamellipodia and filopodia along the fibers (Figure 14). After few days in culture, the small keratinocyte colonies merged into larger colonies progressively forming nearly complete cell monolayer on the top of chitosan nanofibers. When the cells were observed later during the culture, keratinocytes acquired appearance of postconfluent cultures, i.e. the cells retracted and overlapped and a few cells appear apoptotic. These results indicate that electrospun chitosan membranes are a suitable substrate for keratinocyte growth and differentiation, thus appearing as an excellent candidate for engineering wound dressings.
In order to check whether keratinocytes were growing over membranes without penetrating between the fibers, histological staining of vertical sections of paraffin-embedded chitosan membranes cultured with keratinocytes were also performed. These experiments confirm our SEM observations showing that keratinocytes grow over the surface of membranes without penetrating between chitosan fibers (Figure 15), which mimic the in vivo situation where keratinocytes form a covering layer without penetrating in the underlying dermis.

Keratinocyte cultures are characterized by specific expression of several genes, e.g. those encoding keratinocyte differentiating markers, like keratins and involucrin. Proliferative keratinocyte cultures express keratin 5 and keratin 14, which are markers for the undifferentiated cell phenotype. When the differentiation program of keratinocytes begins, the early markers of epidermal differentiation (suprabasal keratin 10 and involucrin) and the later markers of differentiation (filagrin and loricrin) are then expressed [11]. The results of the real-time PCR analysis showed that the expression of keratin 14, a marker of undifferentiated keratinocytes, slowly decreases with the time in culture. Simultaneously with the increase in cell density, we observed an increase in the expression of keratin 10 and involucrin - two of the markers of epidermal differentiation. These results show that epidermal keratinocytes cultured on nanofiber chitosan membranes are able to undergo differentiation as observed in vivo and in vitro on other physiological substrates (Figure 16).

### Example 6: Characterization of normal human fibroblasts and endothelial cells cultured on electrospun chitosan membranes

### 1. METHODS

### 1.1- Materials

A medical grade chitosan of fungi origin (kiOmedine-CsUP™, Kitozyme, Belgium) was used. Its degree of acetylation (DA) and molecular mass (MM) were 19% and 68000, respectively. All chemical and biochemical reagents were analytical grade.

### 1.2- Preparation of chitosan films and electrospun membranes.

Sterile chitosane solutions (1 % in 1 % acetic acid) were obtained by filtration through a 0.22 µm filter.
***Films.*** For obtaining films, adequate volume of sterile chitosan solution were poured in culture dishes and air-dried for 24 h in a laminar flow culture hood under sterile conditions. After complete drying, films were immersed during 2 h in 1 % NaOH in distilled water to neutralise the residual acetic acid. Chitosan films were then rinsed three times in copious amounts of distilled water and dried. Before using to cell culture, the films were equilibrated during 2 h in the culture medium at 37°C.

***Electrospun membranes.*** Membranes formed by electrospun chitosane nanofibers were prepared as described in Example 1. The chitosan membranes were then sterilized in 70% ethanol for 2 hours, washed three times in PBS (30 min each wash) and then equilibrated during 2 h in the culture medium at 37°C.

### 1.3- Cells and cell cultures

Human skin fibroblasts and microvascular endothelial cells (HMEC) were grown culture Petri dishes in Dulbecco's Minimum Essential Medium (DMEM, Lonza) supplemented with 10% fetal bovine serum (FBS, Lonza), antibiotic (100 U/ml penicillin and 100 µg/ml streptomycin, Lonza) and essential amino acid (Lonza) and incubated at 37°C in humidified atmosphere with 5% CO₂. After a confluent cell layer was formed, the cells were detached in PBS containing 0.25% trypsin and 1 mM EDTA and seeded on the various substrates in the same supplemented DMEM medium as described above. The culture medium was changed every 2-3 days.

### 1.4- Cell morphology and proliferation

Cell adhesion, spreading and proliferation were assayed on plastic and on chitosan films and electrospun membranes.

The morphology (adhesion and spreading) of fibroblasts and HMEC was investigated by phase-contrast microscopy or scanning electron microscopy (SEM). Briefly, for the SEM, the chitosan membranes and their adherent cells were harvested and then fixed for 10 min with 2.5% glutaraldehyde solution in Sodium Cacodylate buffer 0.1M pH 7.4, CaCl₂ 0.1% at 4°C. After rinsing three times with Sodium Cacodylate buffer 0.1M pH 7.4 with CaCl₂ 0.1% for 5 min, the specimens were dehydrated in graded ethanol of 25%, 50%, 75%, 95% and 100%, 5 and then 10 min each. The specimens were coated with a thin layer of gold and then subjected to observation by SEM (ktics).

Both the membrane and the film specimens were used for cell morphology and proliferation tests. For proliferation test, the cells were seeded on chitosan films or membranes in 4 cm² polystyrene disc at a density of 10 000 cells/cm². They were then incubated for 1, 3, 5 and 7 days at 37°C in air containing 5% CO₂. WST-1 test was carried out to quantify the viability of the cells which adhered on chitosan at each specified seeding times point and completed by evaluating of proliferation rate using the radioactivity incorporation test. The cells attached on chitosan films or membranes were compared to the cell adhesion observed on the plastic material used as a reference.

### 1.5 Quantification of viable cells and proliferation rate

For measurement of cells proliferation and viability, a colorimetric method was used. This colorimetric assay is based on the cleavage of the tetrazolium salt WST-1 to a formazan-class dye by mitochondrial succinate-tetrazolium reductase in viable cells. As the cells proliferate, more WST-1 is converted to the formazan product. The quantity of formazan dye is directly related to the number of metabolically active cells, and can be quantified by measuring the absorbance at 420-480 nm (Aₘₐₓ 450 nm). Before adding the reagent, the medium was removed and cells culture was washed two times with Dulbecco's. Then, cells were incubated in DMEM with 10% FBS and mixed with WST1 reagent in the ratio 9:1.

After 4h of incubation, 100µl of the supernatant was carefully transferred to 96-well plates and optical density was measured at 450 nm.
The proliferation rate of cells was also measured by evaluating the incorporation of [³H] thymidine into TCA-precipitable DNA.

### 2. RESULTS AND DISCUSSION:

- **Cell adhesion.** The first in vitro experiments were intended to compare the rate of adhesion of human skin fibroblasts and microvascular endothelial cells cultured on plastic, chitosan evaporated films and chitosan electrospun membranes. As compared to evaporated films, ***electrospun nanofibers induced a better and faster attachment*** of both cell types (Figure 17).
- **Cell spreading.** Cells on plastic and chitosan films were easily visualized by phase-contrast microscopy (Figure 18). For cells at the surface of electrospun membranes observation required scanning electron microscopy (SEM) because of a lack of transparency due to the presence of the dense network of nanofibers. Fibroblasts and HMEC in plastic culture dishes are fully spread after one day. As the cultures become confluent, a squeezing of the cells was observed, significant of an excessive proliferation as usually observed in vitro. When seeded on chitosan evaporated films, only few cells were able to attach, confirming our previous data regarding cell adhesion. However, none of them were able to fully spread, as evidenced by comparing the morphology of cells at day 1 on plastic to cells at any time on chitosan films. Moreover, due to the poor adhesion and spreading, these cells tend to form aggregates. These clusters, appearing in our cultures as refringent structures, are an additional evidence of the poor quality of chitosan films as a substrate for cells. In order to confirm that this was not the result of the presence of toxic compounds contaminating the films, floating individual cells and clusters were collected together with the conditioned culture medium and poured in plastic culture dishes (not shown). In these conditions, all the cells (individual or in clusters) attached rapidly and started to proliferate, firmly demonstrating that the poor adhesion and spreading resulted from the inappropriate structure of the chitosan films. On the contrary, cells on electrospun chitosan nanofibers are almost fully spread at day 1 (Figures 19-21), indicating favorable interactions between cells and the nanofiber scaffold. Interesting observations were also made at later time points, showing for example that cell filipodia are in close contact with individual nanofibers and even begin to invade the nanofiber structure and that cells are able to proliferate at a reasonable rate.
- **Cell proliferation.** Microscopic observations were highly suggestive of cell proliferation on chitosan nanofibers but not on chitosan films. This was further investigated by establishing a proliferative index by measurement of the incorporation of [³H] thymidine into TCA-precipitable DNA. As expected from microscopic studies, HMEC and fibroblasts are able to proliferate on chitosane nanofibers (Figures 22). At the contrary a reduction of thymidine incorporation was evidenced for cells on films, as a result of cell detachment and/or cell death because of inappropriate interactions between the substrate (a process named anoïkis).

### 3. DISCUSSION:

Experiments described in this Example 6 have clearly demonstrated that chitosan can be a good substrate for the adhesion, spreading and proliferation of fibroblasts and endothelial cells, but only when manufactured as a scaffold made of nanofibers. Similar data were obtained with keratinocytes (Example 5). These unexpected results may be due to the increased specific surface of the nanofiber biomaterial or to the fact that chitosan nanofibers present structural features that mimic collagen fibers that are the major organic constituent of many tissues in vivo, especially skin, tendons and bones.

These very promising data prompted us to characterize further the biocompatibility and the biological properties of electrospun chitosan in vivo. These experiments are described in Example 7.

### Example 7: Characterization of the biological properties of electrospun chitosan scaffold in vivo

### 1. METHODS

### 1.1- Materials

A medical grade chitosan of fungi origin (kiOmedine-CsUP™, Kitozyme, Belgium) was used. Its degree of acetylation (DA) and molecular mass (MM) were 19% and 68000, respectively. All chemical and biochemical reagents were of analytical grade.

### 1.2- Preparation of chitosan electrospun membranes and chitosan sponges.

Sterile chitosane solutions (1 % in 1 % acetic acid) were obtained by filtration through a 0.22 µm filter.

**Electrospun membranes.** Membranes formed by electrospun chitosane nanofibers were prepared as described in Example 1. The chitosan membranes were then sterilized in 70% ethanol for 2 hours, washed three times in PBS (30 min each wash) and then equilibrated during 2 h in the culture medium at 37°C.

**Sponges.** Due to the absence of three-dimensional structure, evaporated chitosan films were not used in vivo. Instead, chitosan sponges (supplied from Kitozyme) were used. The dried chitosan sponges were then immersed in 70% ethanol solution for sterilization and rinsed many time in large volumes of saline phosphate buffer.

### 1.3- Biocompatibility of chitosan sponges and membranes

All procedures were performed with the approval of the Animal ethical Committee authorities of University of Liège, Belgium. Male Balb/c mice 8-10 weeks old, weighing about 22 g were kept under specific pathogen free (SPF) conditions and given free access to food and water throughout the experiment. The 18 mice were housed in groups of at least six animals. Before implantation, chitosan membranes and sponges were cut into 8.0-mm diameter pieces, sterilised in 70% ethanol and soaked in sterile saline solution. Before starting the experiment the mice received a subcutaneous injection of Temgesic (0.05mg/kg) to prevent pain. This treatment was continued twice daily during ten days after the surgery. The hairs on the backs of the mice were shaved. Anaesthesia was performed by successive intramuscular injections of Domitor (500 µg/kg) and Ketamine (60 mg/kg). Chitosan sponges and electrospun nanofiber membranes were subcutaneously implanted through a 1-cm incision and secured to the inner face of skin with nylon sutures. The mice were waked by intramuscular injection of Antisedan (200 µg/kg). Mice were sacrificed at 1, 2, 4, 8 or 12 weeks after implantation. Serums were collected for the potential presence of anti-chitosan antibodies evaluation. Sponges or electrospun membranes were collected, fixed and used (see below) for histological examination or transmission electronic microscope (TEM).

### 1.4- Preparation of samples for immune-histological examination and TEM

After sacrifice of mice, the chitosan sponges or electrospun membranes were dissected from mice and divided in several equivalent parts. Some were frozen and stored at -80°C for protein and RNA analysis or embedded in Tissue Tek for cryostat sectioning. Another part was fixed in 4% neutralized buffered formaldehyde, embedded in paraffin and processed conventionally to produce 5 µm sections. These sections were then stained (Haematoxylin/eosin, Sirius red, yellow Safran/Haematoxylin) or used for immunostaining. Finally, some pieces were also processed for transmission electron microscopy (TEM) after fixation overnight at 4°C in a 2.5% glutaraldehyde solution (in sodium cacodylate buffer 0.1M, pH 7.4, 0.1% CaCl₂). After rinsing three times in a sodium cacodylate buffer (0.1M; pH 7.4, with CaCl₂ 0.1%) for 5 min, the specimens were further fixed in 1% osmium tetroxide (diluted in 0.1M cacodylate buffer pH 7.4) for 1h at 4°C. The specimens were dehydrated with graduated concentration of ethanol (25%, 50%, 75%, 95% and 100%) and then in propylene oxide. Afterwards, the specimens were infiltrated and embedded in LX112 resin (LADD, USA), polymerized consecutively at 37°C for 24h, 47°C for 24h and 60°C for 48h. Orientation sections for light microscopy were cut at 2 µm thickness and stained with toluidine blue. Ultra-thin sections (50-70 nm) were cut, stained with 4% uranyl acetate (diluted in 50° ethanol), then contrasted with lead citrate and mounted on uncoated grids. Samples were then observed by TEM (FEI Tecnai 10, USA).

### 1.5- Immunostaining

The following primary antibodies were used: (1) monoclonal biotin-conjugated rat anti-mice CD45 (1:1500; PHARMINGEN) for the labelling of leukocytes; (2) polyclonal Guinea pig antibody to vimentin ((1:20; QUARTETT) for the labelling of mesenchymal cells, especially fibroblasts; (3) a "home-made" rabbit anti-mouse type IV collagen (1:100) which identifies basement membrane, here mainly identifying mature blood vessels and (4) mouse anti-alpha smooth muscle actin (1:400; SIGMA) which identifies smooth muscle cells. Before use, paraffin sections were "deparaffined" and rehydrated. Immunohistochemical staining and visualization using diaminobenzidine (DAB) and counter-staining with haematoxylin/eosin for 30 sec.

### 1.6- ELISA assays

96 wells-plates were coated with 180 µg of chitosan solution, incubated for 2 h at room temperature, washed twice with 20 mM Tris buffer (20 min each wash) and then blocked overnight at 4°C by addition of bovine serum albumin (5% in PBS). Plates were then washed 3 times with PBS containing 0.05% of Tween-20. Serum samples from control or from chitosan-implanted mice (sponges or electrospun membranes) were serially diluted for evaluation. Plates were sequentially (i) incubated overnight at 4°C, (ii) washed with PBS/Tween, (iii) incubated for 2 hours with a horseradish peroxidase-conjugated secondary antibody (at a 1/1000 dilution in PBS) and (iv) washed again in PBS/Tween. Staining was obtained by the addition a solution of 1 mM ABTS containing 0.03% H₂O₂ after incubation for 1 h at 37°C in the dark. The reaction was stopped by adding a solution of 1% SDS. The OD at 405 nm was measured and used for comparing the various experimental conditions.

### 2. RESULTS AND DISCUSSION

**Absence of generation of antibody.** Sera were recovered from control mice (that were never in contact with chitosan) or from mice subcutaneously implanted with either electrospun chitosan membranes or chitosan lyophilized sponges. The experimental procedure consisted in an ELISA assay, a standard highly sensitive technique for screening the presence of specific antibodies (Figure 23). The background values obtained from the serum of control mice were considered as being caused by the low affinity existing between chitosan and proteins found in the serum, including immunoglobulins. Measurements performed on sera collected at increasing time after chitosan implantation were always in the range of values observed for control mice, even for long term implanted mice. These data confirm that chitosan, irrespective of its structure (sponge or nanofibers), do not elicit any specific antibody production, thus confirming by this aspect its biocompatibility in vivo.

**Immunohistological examination of implanted chitosan sponges and nanofibers.** Mice were subcutaneously implanted with either electrospun chitosan membranes or chitosan lyophilized sponges. After 1 to 12 weeks, implanted material was recovered, fixed, embedded in paraffin, sectioned and stained by hematoxylin/eosin or by the use of specific antibodies. Chitosan electrospun membranes appear as an undulating dense structure, somehow mimicking the extracellular matrix organization seen in skin in vivo (Figures 24, 25). Cell infiltration and colonization was evidenced and seemed to be time-dependent, starting after less than one week and reaching the center of the biomaterial after 12 to 20 weeks. These cells were shown to be of mesenchymal origin (fibroblasts, myofibroblasts, smooth muscle cells) (Figure 27) and endothelial cells forming structures resembling functional capillaries (Figure 28). A limited number of leukocytes were also detected (Figure 26). As a whole these data are indicative of a physiological progressive remodelling process. By sharp contrast, sponges, that are formed by a multi-lamellae structure containing a large ratio of void space, are not efficiently colonized by living cells and do induce a strong innate immune response (as evidenced by the accumulation of leukocytes and activated mesenchymal cells forming a granuloma at the periphery of the implanted sponges).

**Ultrastructural characterization of implanted biomaterial.** Electrospun chitosan membranes and chitosan lyophilized sponges recovered at increasing time after implantation were also processed and characterized by transmission electron microscopy (TEM).

Chitosan nanofibers appear as black elongated cylindrical structure in longitudinal section and as black circles in cross sections (Figure 29). Numerous fibroblasts were identified within the dense chitosan nanofiber network. These fibroblasts are biosynthetically active since they produce collagen accumulating as physiological fibres and fibrils in close contact with chitosan nanofibers. Some macrophages were also identified, possibly participating to a slow chitosan degradation process (Figure 30). Again, these data strongly suggest a progressive and physiologically ordered remodelling of the nanofiber scaffold.

By contrast, collagen and cell accumulation was found only at the periphery of lyophilized sponges (not shown), confirming immunohistochemical data and illustrating that the nanofibrillar structure of chitosan is crucial for true biocompatibility in vivo.

### A. CONCLUSIONS

Three deeply **unexpected results** were obtained during these in vivo experiments.
- Although the nanofiber network appeared too dense to allow cell migration (see Figure 21), colonization of the entire width (2 mm) of the electrospun membrane was observed within 2 to 3 months. This is due in part to the capacity of cells to change their shape but also to the structure of the scaffold that allows some sliding of any single nanofiber relatively to others because of the absence of reticulation.
- While chitosan sponges are being recognized by the mouse as *"foreign bodies",* and, consequently, encapsulated in a dense capsule (essentially made of collagen, activated mesenchymal cells and leukocytes), electrospun nanofibers are considered as "self' tissue that is progressively filled by host cells and extracellular matrix without any sign of aberrant activation of invading cells. Moreover the limited presence of leukocytes inside the biomaterial is likely to be beneficial for the remodeling process by regulating the properties of other cell types as observed in "in vivo" situation during wound healing.
- The close association observed between chitosan nanofibers, on one side, and cells and the newly deposited extracellular matrix on the other side, illustrates the fact that the design of the structure of the chitosan nanofiber scaffold (rigidity, fiber orientation, fiber density, ...) should strongly influence the properties of the newly formed tissue. This is a crucial importance for any medical application since cell fate and behavior are both strongly regulated by the extracellular environment and since the desired biophysical properties of engineered biomaterials deeply vary according the tissue to be repaired (skin, tendon, bone, etc) .

As an example, the corrugated structure of the electrospun nanofibers, as they are designed in this study, mimics the organization of collagen fibers found in the dermal compartment of the skin. Therefore such biomimetic structure used as progressively degradable scaffold dressing for skin ulcer should favor the optimal repair of skin in terms of elasticity and functionality.

### Example 8: Effect of chitosan nanofibers on wound healing in mice.

### 1. METHODS

### 1.1 Materials

A medical grade chitosan of fungi origin (KiOmedine-CsUP™, Kitozyme, Belgium) was used. Its degree of acetylation (DA) and molecular mass (MM) were 19% and 68000, respectively. All chemical and biochemical reagents were of analytical grade.

### 1.2 Preparation of chitosan electrospun membranes

Sterile chitosan solutions (1% in 1% acetic acid) were obtained by filtration through a 0.22µm filter.

**Electrospun membranes.** Membranes formed by electrospun chitosan nanofibers were prepared as described in example 1. The chitosan membranes were sterilized in 70% ethanol for 2 hours, washed three times in PBS (30 min each wash) and then air-dry for 24 h in a laminar flow culture hood under sterile conditions.

### 1.3 Animal experiments

A total of 30 male Balb/c mice (8-10 weeks) were used, weighing between 20 and 25 g at the time of the experiments. The animal protocols followed in the present study were approved by the Animal Ethical Committee Authorities of University of Liège, Belgium. Before starting the experiment the mice received a subcutaneous injection of Temgesic (0.05 mg/kg) to prevent paint. This treatment was continued twice daily during ten days after the surgery. Mice were individually anesthetized via an intramuscular injection of ketamine (50 mg/kg) and domitor (0.5 mg/kg) for surgery and induction of the excisional wound. The operative skin area was shaved and disinfected using ethanol. Then, the dorsal skin of the animal was excised using a biopsy punch to create an 8-mm wound. The animals were divided into two groups. In group 1, wounds were covered successively with electrospun chitosan membrane, a Tegaderm^{3M} layer and a protective elastic bandage (Elastoblast). In group 2, used as control, wounds were only covered with Tegaderm^{3M} and the elastic bandage (Elastoblast). After surgery, animals were kept in separate cages under specific pathogen free (SPF) conditions and given free access to food and water throughout the experiment. All animals showed good general health conditions throughout the study, as assessed by their weight gain. The animals were sacrificed after 7, 14, and 21 days.

### 1.4 Histological assessment

The material from the skin lesions, either controls or covered first by the electrospun chitosan membrane, was formalin fixed and paraffin embedded for routine histological processing. Five (µm sections obtained from each paraffin block were stained with hematoxylin and eosin (H&E) and evaluated by using a light microscope, or characterized by immunohistochemistry. For the morphological evaluation of skin lesions after H&E staining, three parameters were considered: 1) nature and duration of the inflammatory reaction, 2) composition and thickness of the granulation tissue layer and 3) thickness and quality of the epithelial layer. Immunohistochemical stainings were also performed using various antibodies that allow to monitor the healing rate and the quality of the repair process. These antibodies can recognize as example collagen, fibronectin, CD45 (for leukocytes), alpha-SMA (for smooth muscles cells), KI-67 (for establishing a proliferative index), cytokeratin and involucrine (for keratinocytes).

### 2. RESULTS AND DISCUSSION

The goal of this study was to assess the effect of electospun chitosan on the rate of the healing process and on the quality of the repaired tissue. Macroscopic findings showed that chitosan nanofibers adhered uniformly to the freshly excised wound surface and also absorbed the exudates from the wound surface. The porosity of electrospun chitosan nanofibers promotes gas exchange, which is fundamental for the wound-healing process. No signs of infections were detected in the skin lesion. Standard Hematoxylin and eosin staining of wounds removed from control mice and chitosan treated-mice at 7, 14 and 21 days postwounding are show in Figure 31. On days 7 after injury, histological examination of the wounds treated or not with the electrospun chitosan nanofibers showed that epithelialization process had started on the wound, and the wound bed (dermis) was rich in polynuclear and macrophage inflammatory cells that play crucial role for cleasing the wound and initiating the healing process. Wound treated with electrospun chitosan nanofibers showed a largest extend of infiltration of these inflammatory cells. This indicates that chitosan attracted inflammatory cells during the early phase of wound early without the excessive inflammation. At the 14th postoperative day, wounds were epithelialized but the dermis of control wounds still contained an excessive number of inflammatory cells, showing some delay in the healing process as compared to the wound covered by electrospun chitosan. Moreover, the level of myofibroblastic/fibroblastic cells and the number of newly formed capillaries was higher in presence of electrospun chitosan as compare to controls, again demonstrating a faster healing for treated wounds. At the 21st postoperative day, the epithelium of the electrospun chitosan nanofibers treated wound was thin and well organized resembling to a normal epithelium. The deposition of newly synthesized collagen in the dermis was also well organized with fibrils being oriented parallel to the skin surface, showing an optimal repair of the damaged tissue and indicating that the electrospun chitosan nanofibers increases the rate of healing and help to restore tissue architecture. By contrast the repair process was slower for control wounds.

### CONCLUSIONS

The electrospun chitosan nanofibers used as a wound dressing is fully biocompatible. It showed excellent oxygen permeability and could inhibit exogenous microorganism invasion due to its inherent antimicrobial property of chitosan. Due to its physical form and its chemical structure, electrospun chitosan nanofibers promote the recruitment of inflammatory cells at the early phase of wound healing. Later it increases the rate of blood vessel formation and the maturation of the entire tissue being repaired, including dermis and epidermis, showing that electropun chitosan has many therapeutic advantages and properties when used as a wound dressing.

## Claims

1. A porous layered chitosan scaffold comprising at least two partially fused layers, wherein at least one of the partially fused layer comprises a chitosan nanofiber membrane and the other partially fused layer comprises a chitosan sponge **characterised in that**:
the chitosan nanofiber membrane is electrospun onto the chitosan sponge .

2. The scaffold of claim 1, **characterised in that** the chitosan has a degree of deacetylation between 50 and 100%

3. The scaffold of any one of claims 1 or 2 , wherein chitosan is from fungi origin.

4. The scaffold of any one of claims 1 to 3 , wherein the size of the pores of the porous layered chitosan scaffold ranges from 5·10⁻⁵ to 5·10⁻⁴ m (50 to 500 microns) for the sponge and from 10⁻⁶ to 10⁻⁴ m (1 to 100 microns) for the chitosan nanofiber membrane.

5. The scaffold of any one of claims 1 to 4 , wherein active agents and/or additives selected from the group consisting of proteins, enzymes, complex biological molecules, DNA molecules, RNA molecules, ions, molecules preventing denaturation, misfolding or aggregation of proteins, molecules having antibacterial, antifungal or antiviral properties are incorporated in the nanofiber layer.

6. A process for the preparation of a layered chitosan scaffold of any one of claims 1 to 5 comprising the steps of:
(a) covering an electrospinning collector by a chitosan sponge ;
(b) electrospinning a solution of chitosan onto the sponge covering said collector and collecting a chitosan nanofiber membrane electrospun onto the chitosan sponge ;and
(c) stabilizing the layered chitosan scaffold obtained in step (b) to obtain a partially fused layers chitosan scaffold.

7. The process according to claim 6 further comprising the step of:
(d) freeze-drying of the stabilized scaffold swollen in water for a control of porosity of the nanofiber membrane and/or the sponge.

8. A process according to claim 6 or 7 **characterised in that** the chitosan is co-electrospun in step b with a polymer improving its electrospinning ability.

9. A process according to claim 8 **characterised in that** the polymer is polyethylene oxide.

10. The process of any one of claims 6 to 9, wherein active agents and/or additives selected from the group consisting of proteins, enzymes, complex biological molecules, DNA molecules, RNA molecules, ions, molecules preventing denaturation, misfolding or aggregation of proteins, molecules having antibacterial, antifungal or antiviral properties are co-electrospun with chitosan.

11. The process of any one of claims 6 to 10, further comprising a step wherein the chitosan is partially or totally reacetylated into chitin to get a final degree of acetylation between 50 and 100%

12. The process of claim 11, **characterised in that** chitosan is reactetylated into chitin by incubating said chitosan nanofiber scaffold with acetic anhydride and organic solvents.

13. The layered electrospun chitosanscaffold of any one of claims 1 to 5 or the layered electrospun chitosan scaffold produced by the process of any one of claims 6 to 12 for use as a wound dressing, in regenerative medicine .

## Patentansprüche

1. Poröses schichtartiges Chitosangerüst, das mindestens zwei teilweise fusionierte Schichten umfasst, wobei mindestens eine der teilweise fusionierten Schicht eine Chitoson-Nanofasermembran umfasst und die andere teilweise fusionierte Schicht einen Chitosanschwamm umfasst, **dadurch gekennzeichnet, dass**:
die Chitosan-Nanofasermembran mittels Electrospinning auf den Chitosanschwamm aufgesponnen wird.

2. Gerüst nach Anspruch 1, **dadurch gekennzeichnet, dass** das Chitosan einen Deacetylierungsgrad zwischen 50 und 100% aufweist.

3. Gerüst nach einem der Ansprüche 1 oder 2, wobei das Chitosan pilzlichen Ursprungs ist.

4. Gerüst nach einem der Ansprüche 1 bis 3, wobei die Größe der Poren des porösen schichtarten Chitosangerüsts im Bereich von 5•10⁻⁵ bis 5•10⁻⁴ m (50 bis 500 Mikrometer) für den Schwamm und 10⁻⁶ bis 10⁻⁴ m (1 bis 100 Mikrometer) für die Chitosan-Nanofasermembran beträgt.

5. Gerüst nach einem der Ansprüche 1 bis 4, wobei Wirkstoffe und/oder Additive, ausgewählt aus der Gruppe bestehend aus Proteinen, Enzymen, komplexen biologischen Molekülen, DNA-Molekülen, RNA-Molekülen, Ionen, Molekülen, die den Abbau, die Fehlfaltung oder Aggregation von Proteinen verhindern, Molekülen mit antibakteriellen, antifungalen oder antiviralen Eigenschaften, in die Nanofaserschicht eingearbeitet sind.

6. Verfahren zur Herstellung eines schichtartigen Chitosangerüsts nach einem der Ansprüche 1 bis 5, umfassend die folgenden Schritte:
(a) Bedecken eines Electrospinning-Kollektors mit einem Chitosanschwamm;
(b) Electrospinning einer Chitosanlösung auf den Schwamm, der den Kollektor bedeckt, und Entnehmen einer Chitosan-Nanofasermembran, die mittels Electrospinning auf den Chitosanschwamm aufgesponnen ist; und
(c) Stabilisieren des in Schritt (b) erhaltenen schichtartigen Chitosangerüsts, um ein Chitosangerüst mit partiell fusionierten Schichten zu erhalten.

7. Verfahren nach Anspruch 6, das weiterhin den folgenden Schritt umfasst:
(d) Gefriertrocknen des im Wasser gequollenen stabilisierten Gerüsts für eine Kontrolle der Porosität der Nanofasermembran und/oder des Schwamms.

8. Verfahren nach Anspruch 6 oder 7, **dadurch gekennzeichnet, dass** in Schritt 6 das Chitosan mit einem Polymer, das seine Electrospinning-Fähigkeit verbessert, gemeinsam mittels Electrospinning versponnen wird.

9. Verfahren nach Anspruch 8, **dadurch gekennzeichnet, dass** es sich bei dem Polymer um Polyethylenoxid handelt.

10. Verfahren nach einem der Ansprüche 6 bis 9, wobei Wirkstoffe und/oder Additive, ausgewählt aus der Gruppe bestehend aus Proteinen, Enzymen, komplexen biologischen Molekülen, DNA-Molekülen, RNA-Molekülen, Ionen, Molekülen, die den Abbau, die Fehlfaltung oder Aggregation von Proteinen verhindern, Molekülen mit antibakteriellen, antifungalen oder antiviralen Eigenschaften mit dem Chitosan gemeinsam durch Electrospinning versponnen werden.

11. Verfahren nach einem der Ansprüche des Anspruchs 6 bis 10, das weiterhin einen Schritt umfasst, bei dem das Chitosan teilweise oder vollständig zu Chitin reacetyliert wird, um zu einem Acetylierungsendgrad zwischen 50 und 100% zu gelangen.

12. Verfahren nach Anspruch 11, **dadurch gekennzeichnet, dass** das Chitosan dadurch zu Chitin reacetyliert wird, dass man das Chitosan-Nanofasergerüst mit Essigsäureanhydrid und organischen Lösungsmitteln inkubiert.

13. Schichtartiges durch Electrospinning versponnenes Chitosangerüst nach einem der Ansprüche 1 bis 5 oder schichtartiges durch Electrospinning versponnenes Chitosangerüst, das nach dem Verfahren nach einem der Ansprüche 6 bis 12 produziert wurde, zur Verwendung als Wundverband in der Regenerativmedizin.

## Revendications

1. Support de chitosane en couches poreux comprenant au moins deux couches partiellement fusionnées, dans lequel au moins une des couches partiellement fusionnées comprend une membrane de nanofibres de chitosane et l'autre couche partiellement fusionnée comprend une éponge de chitosane, **caractérisé en ce que** :
la membrane de nanofibres de chitosane est électrofilée sur l'éponge de chitosane.

2. Support de la revendication 1, **caractérisé en ce que** le chitosane a un degré de désacétylation compris entre 50 et 100%

3. Support de l'une quelconque des revendications 1 ou 2, dans lequel le chitosane est d'origine fongique.

4. Support de l'une quelconque des revendications 1 à 3, dans lequel la taille des pores du support de chitosane en couches poreux est comprise dans la gamme des 5.10⁻⁵ à 5.10⁻⁴ m (50 à 500 microns) pour l'éponge et des 10⁻⁶ à 10⁻⁴ m (1 à 100 microns) pour la membrane de nanofibres de chitosane.

5. Support de l'une quelconque des revendications 1 à 4, dans lequel des agents actifs et/ou additifs, choisis dans le groupe constitué par des protéines, enzymes, molécules biologiques complexes, molécules d'ADN, molécules d'ARN, ions, molécules empêchant une dénaturation, un repliement incorrect ou une agrégation de protéines, molécules ayant des propriétés antibactériennes, antifongiques ou antivirales, sont incorporés dans la couche de nanofibres.

6. Procédé pour la préparation d'un support de chitosane en couches de l'une quelconque des revendications 1 à 5, comprenant les étapes consistant à :
(a) recouvrir un collecteur d'électrofilage d'une éponge de chitosane ;
(b) soumettre à un électrofilage une solution de chitosane sur l'éponge recouvrant ledit collecteur et recueillir une membrane de nanofibres de chitosane électrofilée sur l'éponge de chitosane ; et
(c) stabiliser le support de chitosane en couches, obtenu dans l'étape (b), pour obtenir un support de chitosane en couches partiellement fusionnées.

7. Procédé, selon la revendication 6, comprenant en outre l'étape de :
(d) lyophilisation du support stabilisé gonflé dans l'eau pour un contrôle de la porosité de la membrane de nanofibres et/ou de l'éponge.

8. Procédé selon les revendications 6 ou 7, **caractérisé en ce que** le chitosane est électrofilé conjointement, dans l'étape b, avec un polymère améliorant sa capacité d'électrofilage.

9. Procédé selon la revendication 8, **caractérisé en ce que** le polymère est de l'oxyde de polyéthylène.

10. Procédé de l'une quelconque des revendications 6 à 9, dans lequel des agents actifs et/ou additifs, choisis dans le groupe constitué par des protéines, enzymes, molécules biologiques complexes, molécules d'ADN, molécules d'ARN, ions, molécules empêchant une dénaturation, un repliement incorrect ou une agrégation de protéines, molécules ayant des propriétés antibactériennes, antifongiques ou antivirales, sont électrofilés conjointement avec du chitosane.

11. Procédéde l'une quelconque des revendications de revendication 6 à 10, comprenant en outre une étape dans laquelle du chitosane est partiellement ou totalement réacétylé en chitine pour obtenir un degré final d'acétylation compris entre 50 et 100%

12. Procédé de la revendication 11, **caractérisé en ce que** le chitosane est réacétylé en chitine par une incubation dudit support de nanofibres de chitosane avec de l'anhydride acétique et des solvants organiques.

13. Support de chitosane en couches électrofilé de l'une quelconque des revendications 1 à 5, ou support de chitosane en couches électrofilé produit par le procédé de l'une quelconque des revendications 6 à 12, destiné à être utilisé comme pansement pour plaies en médecine régénératrice.
